# EUROPEAN PATENT APPLICATION

(11) **EP 1 070 705 A1**
(43) Date of publication of application: **24.01.2001**
(21) Application number: 99912110.6
(22) Date of filing: 05.04.1999
(51) Int. Cl.: C07D 209/12, C07D 405/10, C07D 409/12, A61K 31/40

(54) **INDOLE DERIVATIVES**

(30) Priority: 06.04.1998 JP 9362598
(71) Applicant: FUJISAWA PHARMACEUTICAL CO., LTD., Osaka-shi Osaka 541-8514 (JP)
(72) Inventor: YAMASAKI, Noritsugu, Himeji-shi Hyogo 672-8071 (JP); IMOTO, Takafumi, Tsukuba-shi Ibaraki 305-0047 (JP); OKU, Teruo, Tsukuba-shi Ibaraki 305-0863 (JP); KAYAKIRI, Hiroshi, Tsukuba-shi Ibaraki 305-0045 (JP); ONOMURA, Osamu, Nagasaki-shi Nagasaki 852-8013 (JP); HIRAMURA, Takahiro, Tsukuba-shi Ibaraki 305-0047 (JP)
(74) Representative: VOSSIUS & PARTNER
(86) International application number: JP9901798
(87) International publication number: WO9951574

(57) **Abstract**

Novel indole derivatives represented by formula (I) or salts thereof, wherein R₁ is aryl-substituted lower alkyl in which the aryl may be substituted by at least one member selected among halogen atoms, aryls, heterocyclic groups, aryl-substituted lower alkyls, aryl-substituted lower alkenyls, lower haloalkyls, (lower cycloalkyl)-substituted lower alkyloxys, (lower cycloalkyloxy)-substituted lower alkyls, aryl-substituted lower alkynyls, aryloxy-substituted lower alkyls, aryl-substituted lower alkyloxys, lower alkylthios, lower alkyloxys, and alkenyls; and R₂ is a lower alkyl, lower alkenyl, aryl, or heterocyclic group optionally substituted by halogeno, lower alkyl, lower alkenyl, or aryl. These compounds have a hypoglycemic activity and a PDE5 inhibitory activity and are useful as a drug.

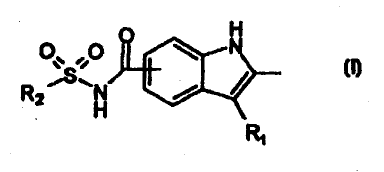

## Description

### TECHNICAL FIELD

The present invention relates to novel indole derivatives, and, more precisely, to novel indole derivatives and their pharmaceutically acceptable salts having blood sugar level-depressing activity or PDE5-inhibiting activity. The present invention also relates to pharmaceutical compositions comprising, as an active ingredient, such indole derivatives or their pharmaceutically acceptable salts.

### DISCLOSURE OF THE INVENTION

The subject matter of the present invention is to provide novel indole derivatives and their pharmaceutically acceptable salts, and also pharmaceutical compositions which comprise, as an active ingredient, such indole derivatives or their pharmaceutically acceptable salts, and which are useful for preventing and treating impaired glucose tolerance, diabetes (type II diabetes), diabetic complications (e.g., diabetic gangrene, diabetic arthropathy, diabetic osteopenia, diabetic glomerulosclerosis, diabetic nephropathy, diabetic dermatopathy, diabetic neuropathy, diabetic cataract, diabetic retinopathy, etc.), syndrome of insulin resistance (e.g., insulin receptor disorders, Rabson-Mendenhall syndrome, leprechaunism, Kobberling-Dunnigan syndrome, Seip syndrome, Lawrence syndrome, Cushing syndrome, acromegaly, etc.), polycystic ovary syndrome, hyperlipidemia, atherosclerosis, cardiovascular disorders (e.g., stenocardia, cardiac failure, etc.), hyperglycemia (e.g., abnormal saccharometabolism such as feeding disorders, etc.), hypertension, pulmonary hypertension, congestive heart failure, glomerulopathy (e.g., diabetic glomerulosclerosis, etc.), tubulointerstitial disorders (e.g., renopathy induced by FK506, cyclosporin, etc.), renal failure, angiostenosis (e.g., after percutaneous arterioplasty), distal angiopathy, cerebral apoplexy, chronic reversible obstructions (e.g., bronchitis, asthma (chronic asthma, allergic asthma)), autoimmune disease, allergic rhinitis, urticaria, glaucoma, diseases characterized by enteromotility disorders (e.g., hypersensitive enteropathy syndrome, etc.), impotence (e.g., organic impotence, psychic impotence, etc.), nephritis, cachexia (e.g., progressive weight loss due to the lipolysis, myolysis, anemia, edema, anorexia, etc. associated with chronic diseases such as cancer, tuberculosis, endocrine disorder, AIDS, etc.), pancreatitis, or restenosis after PTCA.

The present inventors provide a novel indole derivative represented by the formula (I) and its pharmaceutically acceptable salt, and a pharmaceutical composition comprising said compound or its pharmaceutically acceptable salt as an effective ingredient, which is usable for preventing and treating impaired glucose tolerance, diabetes (type II diabetes), diabetic complications (e.g., diabetic gangrene, diabetic arthropathy, diabetic osteopenia, diabetic glomerulosclerosis, diabetic nephropathy, diabetic dermatopathy, diabetic neuropathy, diabetic cataract, diabetic retinopathy, etc.), syndrome of insulin resistance (e.g., insulin receptor disorders, Rabson-Mendenhall syndrome, leprechaunism, Kobberling-Dunnigan syndrome, Seip syndrome, Lawrence syndrome, Cushing syndrome, acromegaly, etc.), polycystic ovary syndrome, hyperlipidemia, atherosclerosis, cardiovascular disorders (e.g., stenocardia, cardiac failure, etc.), hyperglycemia (e.g., abnormal saccharometabolism such as feeding disorders, etc.), hypertension, pulmonary hypertension, congestive heart failure, glomerulopathy (e.g., diabetic glomerulosclerosis, etc.), tubulointerstitial disorders (e.g., renopathy induced by FK506, cyclosporin, etc.), renal failure, angiostenosis (e.g., after percutaneous arterioplasty), distal angiopathy, cerebral apoplexy, chronic reversible obstructions (e.g., bronchitis, asthma (chronic asthma, allergic asthma)), autoimmune disease, allergic rhinitis, urticaria, glaucoma, diseases characterized by enteromotility disorders (e.g., hypersensitive enteropathy syndrome, etc.), impotence (e.g., organic impotence, psychic impotence, etc.), nephritis, cachexia (e.g., progressive weight loss due to the lipolysis, myolysis, anemia, edema, anorexia, etc. associated with chronic diseases such as cancer, tuberculosis, endocrine disorder, AIDS, etc.), pancreatitis, or restenosis after PTCA. 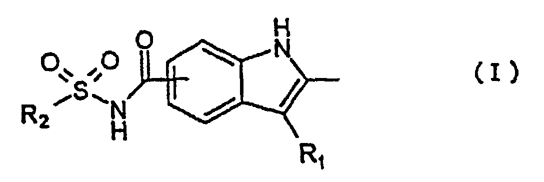 wherein R₁ represents an aryl lower alkyl group, said aryl group may be substituted with one or more groups selected from the group consisting of a halogen atom, an aryl group, a heterocyclic group, an aryl lower alkyl group, an aryl lower alkenyl group, a halo-lower alkyl group, a lower cycloalkyl-lower alkoxy group, a lower cycloalkoxy-lower alkyl group, an aryl lower alkynyl group, an aryloxy lower alkyl group, an aryl lower alkoxy group, a lower alkylthio group, a lower alkoxy group, and an alkenyl group; and R₂ represents a lower alkyl group, a lower alkenyl group, an aryl group, or a heterocyclic group, each of which may be substituted with a hydrogen atom, a lower alkyl group, a lower alkenyl group, or an aryl group.

In the above formula (I), the aryl lower alkyl group presented by R₁ is preferably a halo-aryl lower alkyl group, wherein said aryl group may be substituted with a halo-lower alkyl group, a lower cycloalkyl lower alkoxy group, a lower cycloalkoxy lower alkyl group, an aryl lower alkynyl group, an aryloxy lower alkyl group, a lower alkylthio group, a lower alkoxy group, or a lower alkenyl group.

The indole derivatives provided by the present invention can be prepared according to the following formulae (a) to (c). 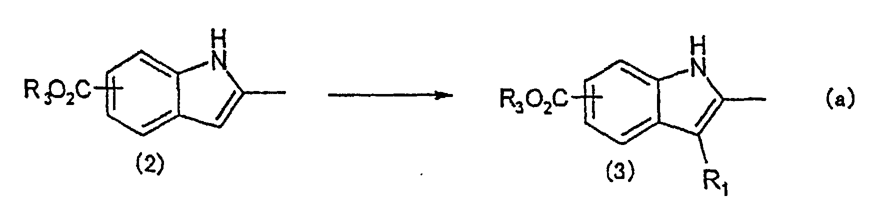 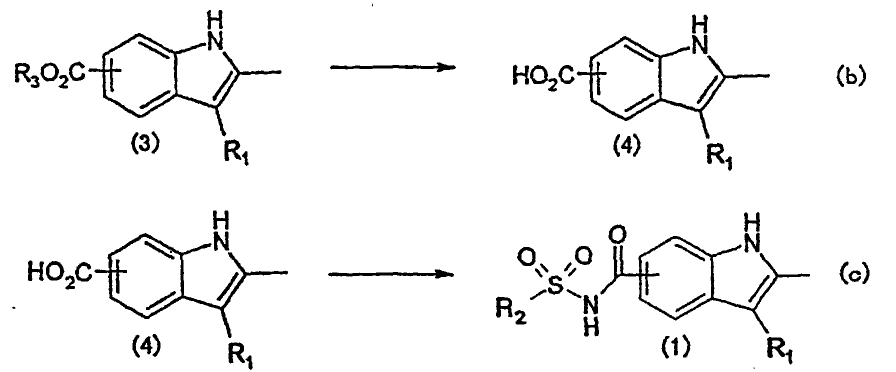 wherein R₁ and R₂ have the same meanings as described above, and R₃ is a lower alkyl group.

Compound (2) can be converted into compound (3) by reacting it with a haloid of R₁ in the presence of silver oxide. Compound (3) can also be obtained by reacting compound (2) with a haloid of R₁ in the presence of tartaric acid and a base such as sodium hydroxide, etc. Further, compound (2) can be converted into compound (3) by reacting it with silanes represented by triethylsilane and aldehydes corresponding to R₁. Compound (4) can be produced by hydrolyzing compound (3) with a base such as lithium hydroxide, sodium hydroxide, potassium hydroxide, etc. Compound (1) can be produced by treating compound (4) with a carboxyl group-activating agent represented by carbonyldiimidazole, 1-(3-(dimethylamino)propyl)-3-ethylcarbodiimide or a salt thereof, dicyclohexylcarbodiimide, isobutyloxycarbonyl chloride, isobutyloyl chloride, pivaloyl chloride, etc., followed by reacting the product with sulfonamide in the presence of a base.

When R₁ in compounds (3), (4), and (1) is an aryl lower-alkyl group, which is substituted by an alkenyl group or an aryl alkenyl group, it is possible to convert the compounds into compounds of which R₁ is an aryl lower-alkyl group, which is substituted by an alkyl group or an aryl alkyl group, by hydrogenating them in the presence of a transition-metal catalyst such as platinum dioxide. Further, when R₁ is an aryl lower-alkyl group, which is substituted by an alkynyl group or an aryl alkynyl group, it is possible to convert the compounds into compounds of which R₁ is an aryl lower-alkyl group, which is substituted by an alkenyl group, an aryl lower-alkenyl group, an alkyl group, or an aryl lower-alkyl group by hydrogenating them in the presence of a transition-metal catalyst such as platinum dioxide.

The indole derivatives of this invention can also be produced according to the following formulae (d) to (j): 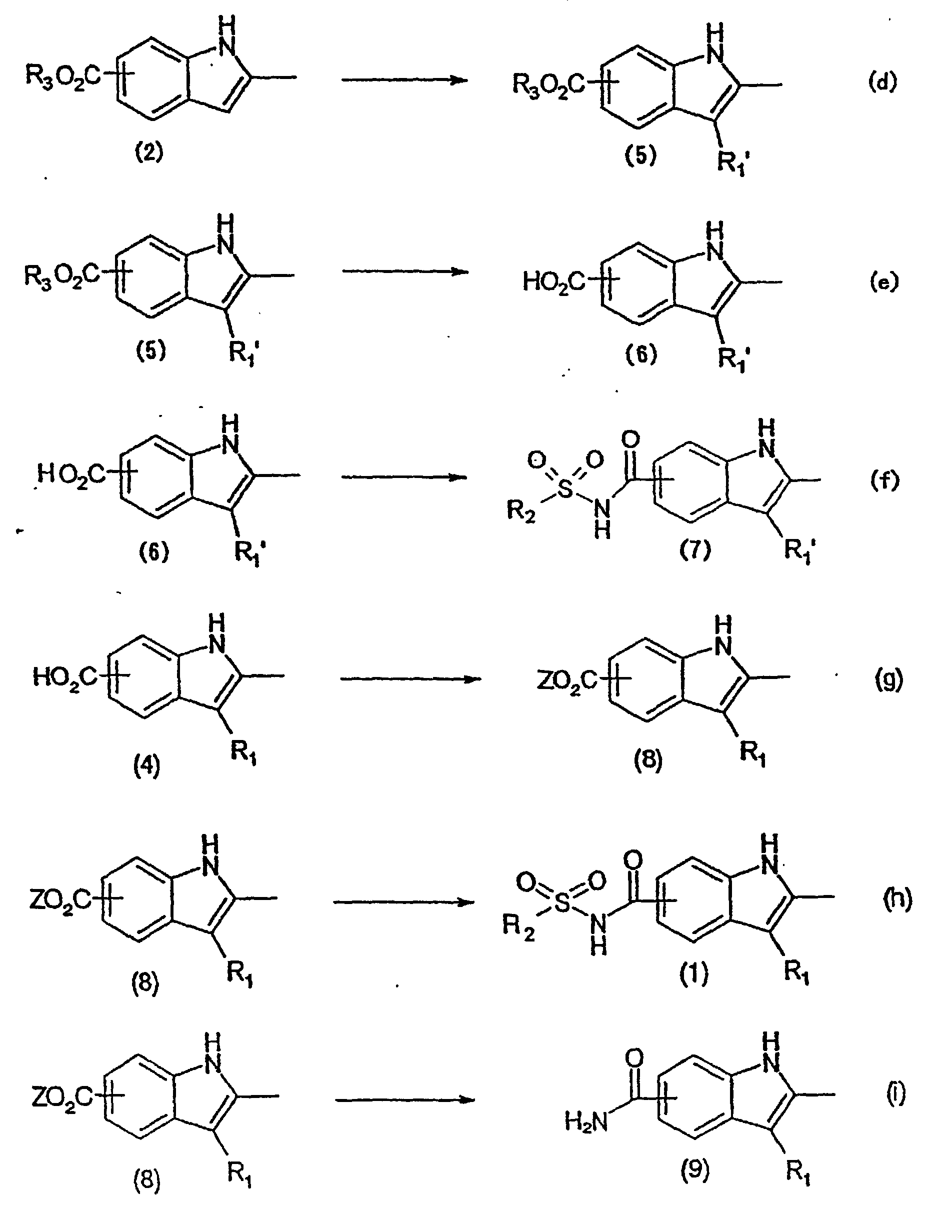 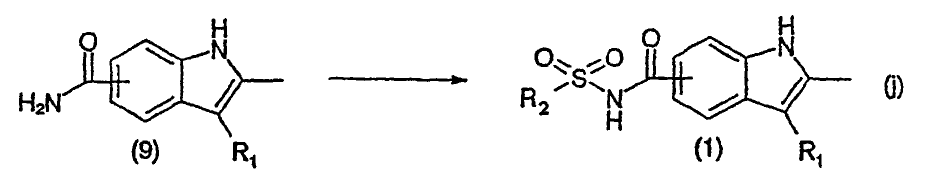 wherein each of R₁, R₂, or R₃ has the same meanings as indicated above; R₁', a halo-aryl lower-alkyl group; and Z, a halogen atom.

Compound (2) can be converted into compound (5) according to formula (d) that is similar to formula (a). Compound (5) can be converted into compound (6) according to formula (e) that is similar to formula (b), and compound (6) can be converted into compound (7) according to formula (f) that is similar to formula (c). Substituent R₁' of compound (5), (6), or (7) can be converted into the above-mentioned substituent R₁. For example, when each of compound (5), (6), and (7) is reacted to aryl borate, thienyl borate, furyl borate, alkene, arylalkene, alkyne or arylalkyne in the presence of a palladium catalyst, the compound can be converted into a compound with an aryl lower-alkyl group, which is equivalent to compound (3), (4), or (1) of which R₁ is substituted by an aryl group, a thienyl group, a furyl group, an alkenyl group, an aryl alkenyl group, an alkynyl group, or an aryl alkynyl group.

Further, compound (4) can be converted into compound (8) by using a halogenating agent such as thionyl chloride, thionyl bromide, phosphorus trichloride, phosphorus pentachloride, phosphorus oxychloride, oxalyl chloride, or phosphorus tribromide (formula(g)). In the formula, Z is a halogen atom, preferably, a bromine atom or a chlorine atom. Compound (1) can be synthesized from compound (8) and sulfonamide in the presence or absence of a base (formula (h)). Compound (9) can be synthesized from compound (8) and ammonia or aqueous ammonia (formula (i)). Compound (1) can be synthesized from compound (9) and sulfonyl halide in the presence or absence of a base (formula (j)).

If desired, the intermediates formed in the above-mentioned steps may optionally be purified, prior to being subjected to the next step, through any conventional purification including, for example, recrystalslization, column chromatography, thin-layer chromatography, high-performance liquid chromatography and the like. If also desired, the final products of the compounds of the present invention may optionally be purified through any conventional purification which is employed in the art of purifying organic compounds and which includes, for example, recrystalslization, column chromatography, thin-layer chromatography, high-performance liquid chromatography and the like. To identify these compounds, employable is any of NMR spectrography, mass spectrography, IR spectrography, elementary analysis, measurement of melting point and others.

Preferred Examples and their details of various definitions as referred to herein to be within the scope of the present invention are described below.

The lower alkyl group used herein preferably has 1 to 6 carbon atoms, including a linear or branched alkyl group such as a methyl group, an ethyl group, an n-propyl group, an i-propyl group, an n-butyl group, an i-butyl group, a sec-butyl group, a t-butyl group, an n-pentyl group, an i-pentyl group, a sec-pentyl group, a t-pentyl group, a 2-methylbutyl group, an n-hexyl group, a 1-methylpentyl group, a 2-methylpentyl group, a 3-methylpentyl group, a 4-methylpentyl group, a 1-ethylbutyl group, a 2-ethylbutyl group, a 1,1-dimethylbutyl group, a 2,2-dimethyl-butyl group, a 3,3-dimethylbutyl group, a 1-ethyl-1-methylpropyl group, an n-hexyl group, etc.

The alkenyl group used herein includes a lower alkenyl group having 2 to 6 carbon atoms and a higher alkenyl group having 7 to 20 carbon atoms, and examples thereof include a linear or branched alkenyl group, such as a vinyl group, an ethenyl group, a 1-propenyl group, a 2-propenyl group, a 1-butenyl group, a 2-butenyl group, a 3-butenyl group, a 1,3-butadienyl group, a 1-pentenyl group, a 2-pentenyl group, a 3-pentenyl group, a 4-pentenyl group, a 1-hexenyl group, a 2-hexenyl group, a 3-hexenyl group, a 4-hexenyl group, a 5-hexenyl group, a 1,4-methylpentenyl group, a 1-heptenyl group, a 1-octenyl group, a 1-nonenyl group, a 1-decenyl group, a 1-undecenyl group, a 1-dodecenyl group, a 1-tridecenyl group, a 1-tetradecenyl group, a 1-pentadecenyl group, a 1-hexadecenyl group, a 1-octadecenyl group, etc. Preferably, those having 2 to 8 carbon atoms are used.

The lower alkenyl group preferably includes vinyl, ethenyl, 1-propenyl, 2-propenyl, 1-butenyl, 2-butenyl, 3-butenyl, 1,3-butadienyl, 1-pentenyl, 2-pentenyl, 3-pentenyl, 4-pentenyl, 1-hexenyl, 2-hexenyl, 3-hexenyl, 4-hexenyl, 5-hexenyl, 1,4-methylpentenyl, etc.

The aryl group means those having 6 to 10 carbon atoms such as phenyl, naphthyl, and such. When simply referred to as "naphthyl group", it includes 1-naphthyl and 2-naphthyl groups.

The aryl lower alkyl group means the lower alkyl group described above to which the above-described aryl group is bonded, including benzyl, 1-phenylethyl, 2-phenylethyl, phenylpropyl, phenylbutyl, phenylpentyl, phenylhexyl, naphthylmethyl, naphthylethyl, naphthylpropyl, naphthylbutyl, naphthylpentyl, naphthylhexyl, etc.

The halogen atom includes fluorine, chlorine, bromine, and iodine atoms.

The heterocyclic group means an unsaturated monocyclic or polycyclic heterocyclic group containing at least one hetero atom such as oxygen, sulfur, and nitrogen atoms, including furanyl, thiophenyl, pyrrolyl, imidazolyl, furyl, thienyl, thiazolyl, pyridyl, benzimidazolyl, benzofuryl, indolyl, benzothienyl, quinolyl, isoquinolyl, etc. The position of the substituted hetero atom described above on the aromatic ring is not particularly restricted.

The aryl lower alkenyl group means the above-described lower alkenyl group to which the above-described aromatic group is bonded, including 1-phenylethenyl, 2-phenylethenyl, 1-phenyl-1-propenyl, 2-phenyl-1-propenyl, 3-phenyl-1-propenyl, 1-phenyl-2-propenyl, 2-phenyl-2-propenyl, 3-phenyl-2-propenyl, 1-phenyl-1-butenyl, 2-phenyl-1-butenyl, 4-phenyl-2-butenyl, 3-phenyl-2-propenyl, 2-phenyl-1-pentenyl, 2-phenyl-3-pentenyl, 2-phenyl-1-pentenyl, 2-phenyl-1-hexenyl, etc.

The halo-lower alkyl group means the above-described lower alkyl group substituted with the above-described halogen atom, including fluoromethyl, difluoromethyl, trifluoromethyl, chloromethyl, dichloromethyl, trichloromethyl, bromomethyl, dibromomethyl, tribromomethyl, iodomethyl, 1-fluoroethyl, 1-chloromethyl, 1-bromomethyl, 2-fluoroethyl, 2-chloromethyl, 2-bromomethyl, 1,1-difluoroethyl, 1,1-dichloroethyl, 1,1-dibromoethyl, 2,2-difluoroethyl, 2,2-dichloroethyl, 2,2-dibromoethyl, 1,2-difluoroethyl, 1,2-dichloroethyl, 1,2-dibromoethyl, 2,2,2-trifluoroethyl, heptafluoroethyl, 1-fluoropropyl, 1-chloropropyl, 1-bromopropyl, 2-fluoropropyl, 2-chloropropyl, 2-bromopropyl, 3-fluoropropyl, 3-chloropropyl, 3-bromopropyl, 1,1-difluoropropyl, 1,1-dichloropropyl, 1,1-dibromopropyl, 1,2-difluoropropyl, 1,2-dichloropropyl, 1,2-dibromopropyl, 2,3-difluoropropyl, 2,3-dichloropropyl, 2,3-dibromopropyl, 3,3,3-trifluoropropyl, 2,2,3,3,3-pentafluoropropyl, 2-fluorobutyl, 2-chlorobutyl, 2-bromobutyl, 4-fluorobutyl, 4-chlorobutyl, 4-bromobutyl, 4-iodobutyl, 3,4-dichlorobutyl, 2,4-dibromopentyl, 4,4,4-pentafluorobutyl, 2,2,3,3,4,4,4-heptafluorobutyl, perfluorobutyl, 2-fluoropentyl, 2-chloropentyl, 2-bromopentyl, 5-fluoropentyl, 5-chloropentyl, 3-iodopentyl, 5-bromopentyl, 2-fluorohexyl, 2-chlorohexyl, 2-bromohexyl, 6-fluorohexyl, 6-chlorohexyl, 6-bromohexyl, 1,3,5-trifluorohexyl, perfluorohexyl, etc.

The lower alkoxy group means a straight or branched alkoxyl group having up to 6 carbon atoms, including methoxy, ethoxy, n-propyloxy, i-propyloxy, n-butyloxy, i-butyloxy, sec-butyloxy, t-butyloxy, n-pentyloxy, i-pentyloxy, sec-pentyloxy, 2,2-dimethylpropyloxy, 2-methylbutoxy, n-hexyloxy, i-hexyloxy, t-hexyloxy, sec-hexyloxy, 2-methylpentyloxy, 3-methylpentyloxy, 1-ethylbutyloxy, 2-ethylbutyloxy, 1,1-dimethylbutyloxy, 2,2-dimethylbutyloxy, 3,3-dimethylbutyloxy, 1-ethyl-1-methylpropyloxy, etc.

The lower cycloalkyl-lower alkoxy group means the above-described lower alkoxy group to which a cycloalkyl group having 3 to 7 carbon atoms is bonded. Such a cycloalkyl group includes cyclopropyl, cyclobutyl, cyclopentyl, cyclohexyl, cycloheptyl, and such. Examples of the lower cycloalkyl-lower alkoxy group include (cyclopropylmethyl)oxy, (2-cyclopropylethyl)oxy, (cyclobutylmethyl)oxy, (3-cyclobutylpropyl)oxy, (cyclopentylmethyl)oxy, (2-cyclopentylethyl)oxy, (4-cyclopentylbutyl)oxy, (cyclohexylmethyl)oxy, (1-cyclohexylethyl)oxy, (2-cyclohexylethyl)oxy, (3-cyclohexylpropyl)oxy, (2-cyclohexylpropyl)oxy, (1-cyclohexylpropyl)oxy, (4-cyclohexylbutyl)oxy, (3-cyclohexylbutyl)oxy, (2-cyclohexylbutyl)oxy, (6-cyclohexylhexyl)oxy, (1-cyclohexylbutyl)oxy, cycloheptylmethyloxy, etc.

The lower cycloalkoxy-lower alkyl group means the above-described lower alkyl group having bonded thereto a cycloalkoxy group having 3 to 7 carbon atoms, for example, cyclopropyloxy, cyclobutyloxy, cyclopentyloxy, cyclohexyloxy, cycloheptyloxy, and such. Examples thereof include (cyclopropyloxy)methyl, 2-(cyclopropyloxy)ethyl, (cyclobutyloxy)methyl, 3-(cyclobutyloxy)propyl, cyclopentyl-oxymethyl, 2-(cyclopentyloxy)ethyl, 4-(cyclopentyloxy)butyl, (cyclohexyloxy)methyl, 1-(cyclohexyloxy)ethyl, 2-(cyclohexyloxy)ethyl, 3-(cyclohexyloxy)propyl, 2-(cyclohexyloxy)propyl, 1-(cyclohexyloxy)propyl, 4-(cyclohexyloxy)butyl, 3-(cyclohexyloxy)butyl, 2-(cyclohexyloxy)butyl, 6-(cyclohexyloxy)hexyl, 1-(cyclohexyloxy)butyl, (cycloheptyloxy)methyl, etc.

The aryl lower alkynyl group means an alkynyl group having 2 to 6 carbon atoms to which the above-described aryl group is bonded, including phenylethynyl, 3-phenyl-1-propynyl, 3-phenyl-1-butynyl, 4-phenyl-1-butynyl, 4-phenyl-2-butynyl, 1-phenyl-2-pentynyl, 1-phenyl-4-pentynyl, 6-phenyl-1-bexynyl, etc.

The aryloxy lower alkyl group means the above-described aryl group to which the above-described lower alkyl group is bonded via an oxygen atom, including (phenyloxy)methyl, (1-naphthyloxy)methyl, (2-naphthyloxy)methyl, 1-(phenyloxy)ethyl, 2-(phenyloxy)ethyl, 1-(1-naphthyloxy)ethyl, 1-(2-naphthyloxy)ethyl, 2-(1-naphthyloxy)ethyl, 2-(2-naphthyloxy)ethyl, 1-(phenyloxy)propyl, 2-(phenyloxy)propyl, 3-(phenyloxy)propyl, 1-(1-naphthyloxy)propyl, 1-(2-naphthyloxy)propyl, 2-(1-naphthyloxy)propyl, 2-(2-naphthyloxy)propyl, 3-(1-naphthyloxy)propyl, 3-(2-naphthyloxy)propyl, 4-(phenyloxy)butyl, 5-(phenyloxy)pentyl, 6-(phenyloxy)hexyl, etc.

The aryl lower alkoxy group means the above-described aryl group to which the above-described lower alkoxy group is bonded, including benzyloxy, 1-naphthylmethyloxy, 2-naphthylmethyloxy, (1-phenylethyl)oxy, (2-phenylethyl)oxy, (1-naphthylethan-1-yl)oxy, (2-naphthylethan-1-yl)oxy, (1-naphthylethan-2-yl)oxy, (2-naphthylethan-2-yl)oxy, (1-phenylpropyl)oxy, (2-phenylpropyl)oxy, (3-phenylpropyl)oxy, (1-naphthylpropan-1-yl)oxy, (2-naphthylpropan-1-yl)oxy, (1-naphthylpropan-2-yl)oxy, (2-naphthylpropan-2-yl)oxy, (1-naphthylpropan-3-yl)oxy, (2-naphthylpropan-3-yl)oxy, (4-phenylbutyl)oxy, (2-naphthylbutan-4-yl)oxy, (5-phenylpentyl)oxy, (2-naphthylpentan-5-yl)oxy, (6-phenylhexyl)oxy, (1-naphthylhexan-6-yl)oxy, etc.

The lower alkylthio group means a straight or branched alkylthio group having up to 6 carbon atoms, including methylthio, ethylthio, n-propylthio, i-propylthio, n-butylthio, i-butylthio, sec-butylthio, t-butylthio, n-pentylthio, i-pentylthio, sec-pentylthio, t-dimethylpropylthio, 2-methylbutylthio, n-hexylthio, i-hexylthio, t-hexylthio, sec-hexylthio, 2-methylpentylthio, 3-methylpentylthio, 1-ethylbutylthio, 2-ethylbutylthio, 1,1-dimethylbutylthio, 2,2-dimethylbutylthio, 3,3-dimethylbutylthio, 1-ethyl-1-methylpropylthio, etc. Preferred are those having carbon atoms 1 to 4 such as methylthio, ethylthio, n-propylthio, i-propylthio, n-butylthio, i-butylthio, sec-butylthio, t-butylthio, and such.

The halo-aryl group means the above-described aryl group substituted with the above-described halogen atom, including 2-fluorophenyl, 2-chlorophenyl, 2-bromophenyl, 2-iodophenyl, 3-fluorophenyl, 3-chlorophenyl, 3-bromophenyl, 3-iodophenyl, 4-fluorophenyl, 4-chlorophenyl, 4-bromophenyl, 4-iodophenyl, 2,3-dichlorophenyl, 2,4-dichlorophenyl, 2,5-dichlorophenyl, 2,6-dichlorophenyl, 4-bromo-2-chlorophenyl, 1-bromonaphthalen-2-yl, 2-chloronaphthalen-1-yl, 5-chloronaphthalen-1-yl, 6-chloronaphthalen-1-yl, 4-chloroisoquinolin-8-yl, 2-chloroquinolin-4-yl, 4-bromoisoquinolin-1-yl, 5-chlorothiophen-2-yl, 5-bromothiophen-2-yl, 5-chlorothiophen-3-yl, etc.

Preferred salts of the indole derivatives of the present invention are non-toxic, ordinary pharmaceutically acceptable salts thereof. For example, mentioned are salts of the derivatives with bases as well as acid-addition salts of the derivatives, which include, for example, salts thereof with inorganic bases, such as salts with alkali metals (e.g., sodium, potassium); salts with alkaline earth metals (e.g., calcium, magnesium); ammonium salts; salts with organic amines (e.g., triethylamine, pyridine, picoline, ethanolamine, triethanolamine, dicyclohexylamine, N,N'-dibenzylethylenediamine); salts with inorganic acids (e.g., hydrochloric acid, hydrobromic acid, sulfuric acid, phosphoric acid); salts with organic carboxylic acids (e.g., formic acid, acetic acid, trifluoroacetic acid, maleic acid, tartaric acid); salts with sulfonic acids (e.g., methanesulfonic acid, benzenesulfonic acid, p-toluenesulfonic acid); salts with basic or acidic amino acids (e.g., arginine, aspartic acid, glutamic acid), etc.

The compounds of the invention could contain one or more chiral centers, therefore they could be enantiomers or diastereomers. Few of the compounds containing alkenyl group could also be cis- or trans-isomers. In both cases, each of such isomers as well as the mixture thereof are within the scope of this invention.

The compounds of the invention can also exist as tautomers, and individual of such tautmers and the mixture thereof are within the scope of this invention.

The compounds of the invention and their salts can be solvate, which are also within the invention. The solvent for the solvate is preferably hydrate or ethanol.

Specific examples of the inventive compound are 3-(2-chloro-4-(t-butylthio)benzyl)-2-methyl-5-(1-pentanesulfonylcarbamoyl)indole, 3-(2-chloro-4-(t-butylthio)benzyl)-2-methyl-5-(4-methylbenzene)sulfonylcarbamoyl)indole, 3-(2-chloro-4-iodobenzyl)-2-methyl-5-(1-pentanesulfonylcarbamoyl)indole, 3-(2-chloro-4-iodobenzyl)-2-methyl-5-((4-methyl-benzene)sulfonylcarbamoyl)indole, 3-(2-chloro-4-(phenylethynyl)benzyl)-2-methyl-5-(1-pentanesulfonylcarbamoyl)indole, 3-(2-chloro-4-(phenylethynyl)benzyl)-2-methyl-5-((4-methylbenzene)sulfonylcarbamoyl)- indole, 3-(2-chloro-4-(2-phenylethenyl)benzyl)-2-methyl-5-((4-methylbenzene)sulfonylcarbamoyl)indole, 3-(2-chloro-4-(2-phenylethenyl)benzyl)-2-methyl-5-(1-pentanesulfonylcarbamoyl)indole, 3-(2-chloro-4-(2-phenylethyl)benzyl)-2-methyl-5-((4-methylbenzene)sulfonylcarbamoyl)indole, 3-(2-chloro-4-(benzyloxy)-benzyl)-2-methyl-5-((4-methylbenzene)sulfonylcarbamoyl)indole, 3-(2-chloro-4-(cyclohexylmethyloxy)benzyl)-2-methyl-5-((4-methylbenzene)sulfonylcarbamoyl)indole, 3-(2-chloro-4-phenylbenzyl)-5-((5-chloro-2-thiophenesulfonyl)carbamoyl)-2-methylindole, 3-(2-chloro-4-phenylbenzyl)-5-((5-bromo-2-thiophenesulfonyl)carbamoyl)-2-methylindole, 3-(2-chloro-4-phenylbenzyl)-2-methyl-5-(4-pentenesulfonylcarbamoyl)indole, 3-((1-bromonaphthalen-2-yl)methyl)-5-((5-chloro-2-thiophenesulfonyl)-carbamoyl)-2-methylindole, 3-((1-bromonaphthalen-2-yl)methyl)-5-((5-bromo-2-thiophenesulfonyl)carbamoyl)-2-methylindole, 3-(4-bromo-2-chlorobenzyl)-2-methyl-5-((4-methylbenzene)sulfonylcarbamoyl)indole, 3-(4-bromo-2-chlorobenzyl)-2-methyl-5-((4-vinylbenzene)sulfonylcarbamoyl)indole, 3-(4-bromo-2-chlorobenzyl)-2-methyl-5-((2-phenylethenyl)sulfonylcarbamoyl)indole, 3-(4-bromo-2-chlorobenzyl)-2-methyl-5-((1-pentene)sulfonylcarbamoyl)indole, 3-(4-bromo-2-chlorobenzyl)-5-((5-bromo-2-thiophenesulfonyl)carbamoyl)-2-methylindole, 3-(4-bromo-2-chlorobenzyl)-2-methyl-5-(4-pentenesulfonylcarbamoyl)indole, 5-((5-chloro-2-thiophenesulfonyl)carbamoyl)-3-(2,4-dichlorobenzyl)-2-methylindole, 5-((5-bromo-2-thiophenesulfonyl)-carbamoyl)-3-(2,4-dichlorobenzyl)-2-methylindole, 3-(2-chloro-4-(trifluoromethyl)benzyl)-2-methyl-5-(1-pentanesulfonylcarbamoyl)indole, 3-(2-chloro-4-(trifluoromethyl)benzyl)-2-methyl-5-(4-methylbenzenesulfonylcarbamoyl)indole, 3-(2-chloro-4-(trifluoromethyl)benzyl)-2-methyl-5-((5-chloro-2-thiophenesulfonyl)carbamoyl)indole, 3-(2-chloro-4-(trifluoromethyl)-benzyl)-2-methyl-5-((5-bromo-2-thiophenesulfonyl)carbamoyl)-indole, 3-(2-chloro-4-(trifluoromethyl)benzyl)-2-methyl-5-((4-vinylbenzene)sulfonylcarbamoyl)indole, 3-(2-chloro-4-(trifluoromethyl)benzyl)-2-methyl-5-((2-phenylethenyl)sulfonylcarbamoyl)- indole, 3-(2-chloro-4-(trifluoromethyl)benzyl)-2-methyl-5-((1-pentene)sulfonylcarbamoyl)indole, 3-(2-chloro-4-(phenoxymethyl)-benzyl)-2-methyl-5-(1-pentanesulfonylcarbamoyl)indole, 3-(2-chloro-4-(phenoxymethyl)benzyl)-2-methyl-5-(4-methylbenzenesulfonylcarbamoyl)indole, 3-(2-chloro-4-(cyclohexyloxymethyl)-benzyl)-2-methyl-5-(1-pentanesulfonylcarbamoyl)indole, 3-(2-chloro-4-(cyclohexyloxymethyl)benzyl)-2-methyl-5-(4-methylbenzenesulfonylcarbamoyl)indole, 3-(2-chloro-4-ethoxybenzyl)-2-methyl-5-(4-methylbenzenesulfonylcarbamoyl)indole, 3-(2-chloro-4-ethoxybenzyl)-2-methyl-5-(1-pentanesulfonylcarbamoyl)indole, 3-(2-chloro-4-(thiophen-2-yl)benzyl)-2-methyl-5-(4-methylbenzenesulfonylcarbamoyl)indole, 3-(2-chloro-4-(thiophen-2-yl-)benzyl)-2-methyl-5-(1-pentanesulfonylcarbamoyl)indole, 3-(2-chloro-4-(furan-2-yl)benzyl)-2-methyl-5-(1-pentanesulfonylcarbamoyl)indole, 3-(2-chloro-4-(furan-2-yl)benzyl)-2-methyl-5-(4-methylbenzenesulfonylcarbamoyl)indole, 3-(2-chloro-4-(1-hexen-2-yl)benzyl)-2-methyl-5-(4-methylbenzenesulfonylcarbamoyl)indole, 3-(2-chloro-4-(1-hexen-1-yl)benzyl)-2-methyl-5-(4-methylbenzenesulfonylcarbamoyl)indole, 3-(2-chloro-4-(1-hexen-2-yl)benzyl)-2-methyl-5-(1-pentanesulfonylcarbamoyl)indole, 3-(2-chloro-4-(1-hexen-1-yl)benzyl)-2-methyl-5-(1-pentanesulfonylcarbamoyl)indole, etc.

The indole derivatives and their pharmaceutically acceptable salts of the present invention that are mentioned hereinabove are effective for preventing and treating various disorders, for example, impaired glucose tolerance, diabetes (type II diabetes), diabetic complications (e.g., diabetic nephropathy, diabetic neuropathy, diabetic retinopathy, etc.), syndrome of insulin resistance (e.g., insulin receptor disorders, Rabson-Mendenhall syndrome, leprechaunism, Kobberling-Dunnigan syndrome, Seip syndrome, Lawrence syndrome, Cushing syndrome, acromegaly, etc.), polycystic ovary syndrome, hyperlipidemia, atherosclerosis, cardiovascular disorders (e.g., stenocardia, cardiac failure, etc.), hyperglycemia (e.g., abnormal saccharometabolism such as feeding disorders, etc.), and hypertension based on their blood sugar level-depressing activity, as well as stenocardia, hypertension, pulmonary hypertension, congestive heart failure, glomerulopathy (e.g., diabetic glomerulosclerosis, etc.), tubulointerstitial disorders (e.g., renopathy induced by FK506, cyclosporin, etc.), renal failure, atherosclerosis, angiostenosis (e.g., after percutaneous arterioplasty), distal angiopathy, cerebral apoplexy, chronic reversible obstructions (e.g., bronchitis, asthma (chronic asthma, allergic asthma), etc.), autoimmune diseases, allergic rhinitis, urticaria, glaucoma, diseases characterized by enteromotility disorders (e.g., hypersensitive enteropathy syndrome, etc.), impotence (e.g., organic impotence, psychic impotence, etc.), diabetic complications (e.g., diabetic gangrene, diabetic arthropathy, diabetic glomerulosclerosis, diabetic dermatopathy, diabetic neuropathy, diabetic cataract, diabetic retinopathy, etc.), nephritis, cachexia (e.g., progressive weight loss due to the lipolysis, myolysis, anemia, edema, anorexia, etc. associated with chronic diseases such as cancer, tuberculosis, endocrine disorder, AIDS, etc.), pancreatitis, and restenosis after PTCA based on their cGMP-PDE (especially PDE5)-inhibiting activity, smooth muscle relaxing activity, bronchodilating activity, vasodilating activity, smooth muscle cell suppressing activity, and antiallergic activity.

To use the indole derivatives of the present invention for treating diseases or disorders such as those mentioned hereinabove, they may be formulated into pharmaceutical compositions of ordinary forms, which comprise, as an active ingredient, any of the derivatives along with pharmaceutically acceptable carriers, such as organic or inorganic solid or liquid vehicles, and which are suitable for oral administration, parenteral administration, or external application. The pharmaceutical compositions may be of any solid form of tablets, granules, powders, capsules, etc., or may be of any liquid form of solutions, suspensions, syrups, emulsions, lemonades, etc.

If desired, the pharmaceutical compositions may further contain a pharmaceutical aid, a stabilizer, a wetting agent, and also any ordinary additive of, for example, lactose, citric acid, tartaric acid, stearic acid, magnesium stearate, terra alba, sucrose, corn starch, talc, gelatin, agar, pectin, peanut oil, olive oil, cacao butter, ethylene glycol, etc.

The amount of the above-mentioned derivative of the present invention to be used shall vary, depending on the age and the condition of patients, the type and the condition of diseases or disorders, and the type of the derivative to be used. In general, for oral administration, the dose of the derivative may be from 1 to 100 mg/kg; and for intramuscular injection or intravenous injection, it may be from 0.1 to 10 mg/kg. Such a unit dose may be applied to a patient once to four times a day.

### BRIEF DESCRIPTION OF THE DRAWINGS

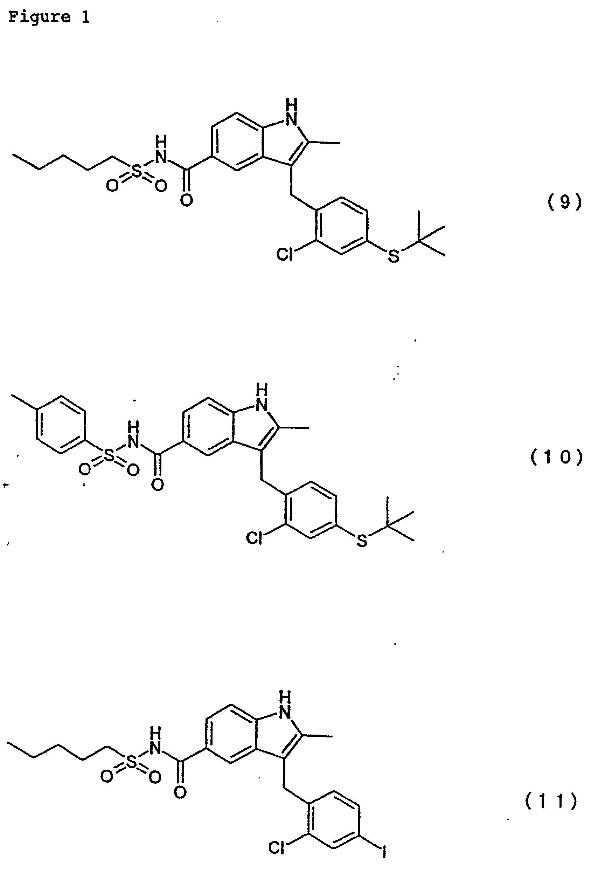

Fig. 1 shows chemical formulae of compound (9) to compound (11).

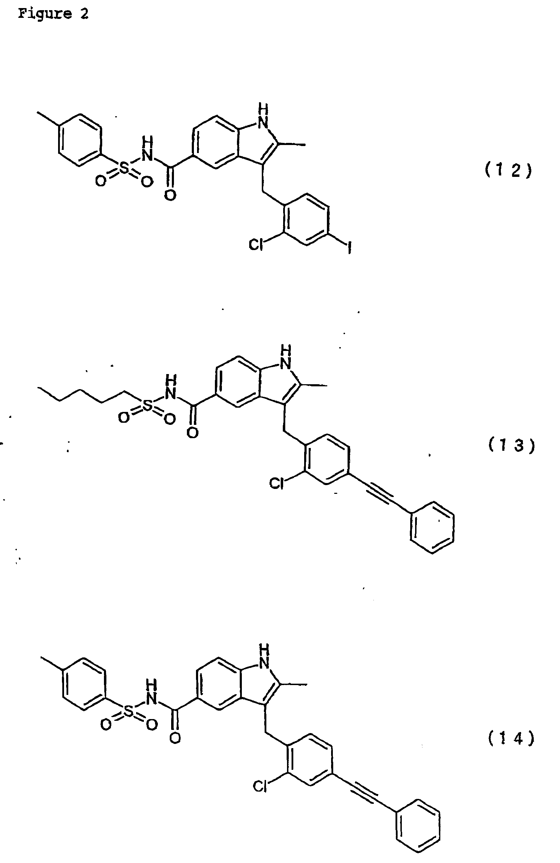

Fig. 2 shows chemical formulae of compound (12) to compound (14).

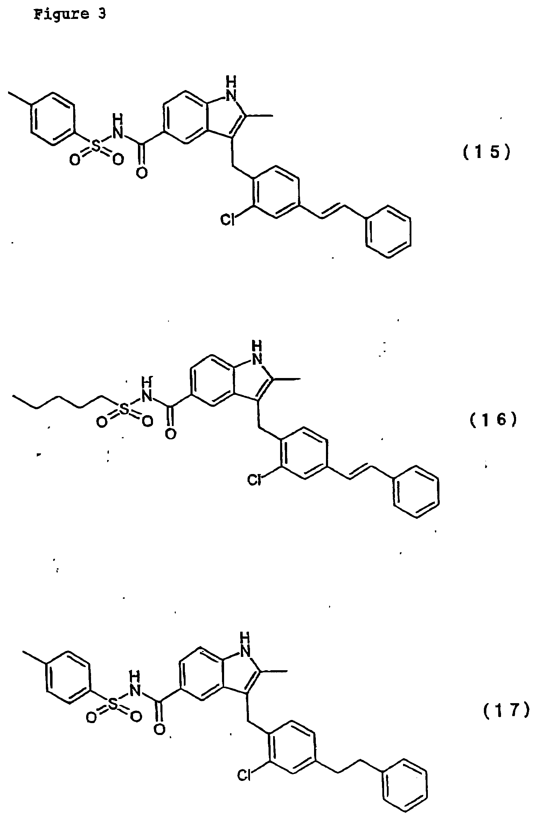

Fig. 3 shows chemical formulae of compound (15) to compound (17).

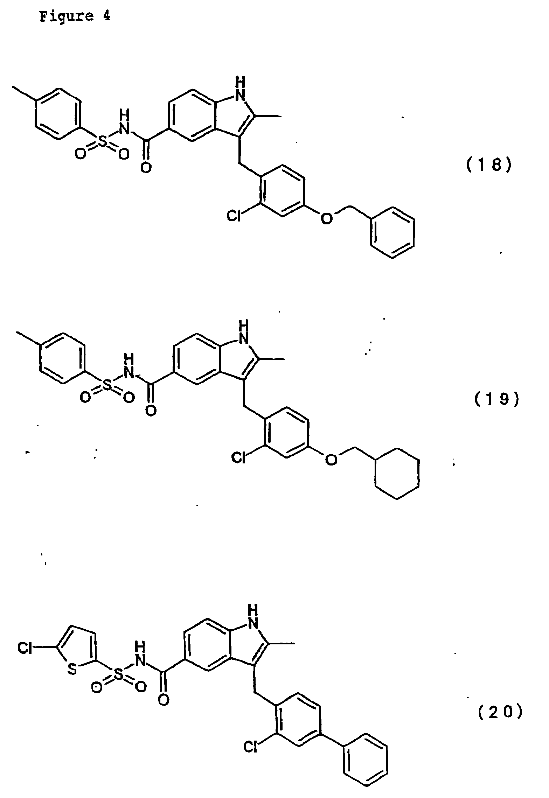

Fig. 4 shows chemical formulae of compound (18) to compound (20).

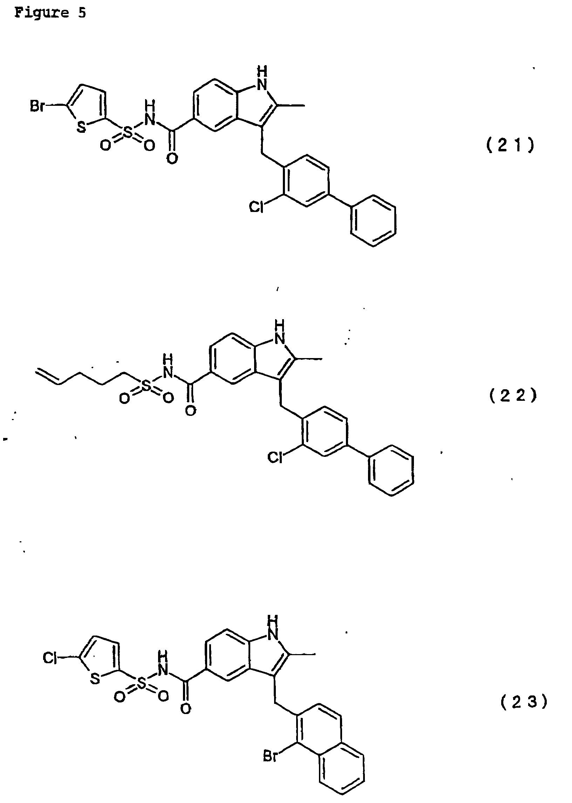

Fig. 5 shows chemical formulae of compound (21) to compound (23).

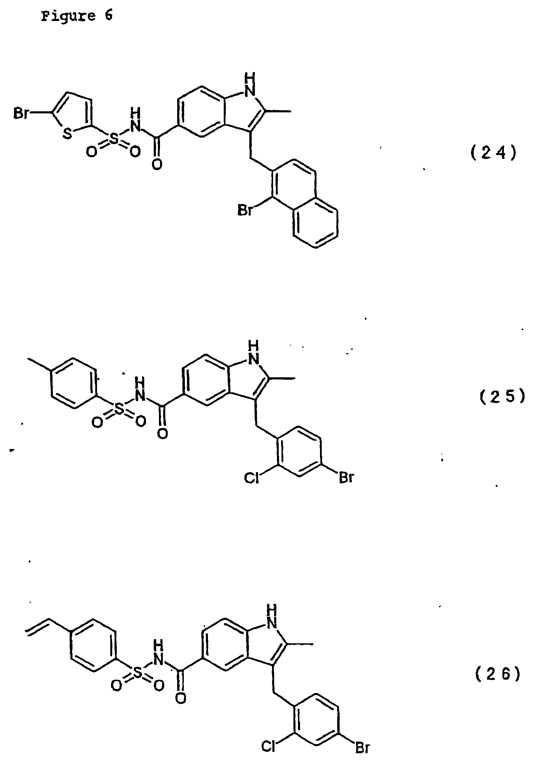

Fig. 6 shows chemical formulae of compound (24) to compound (26).

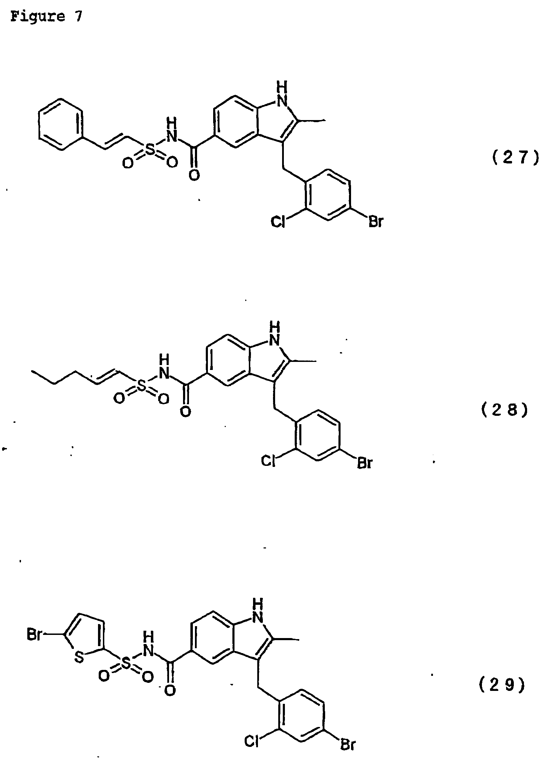

Fig. 7 shows chemical formulae of compound (27) to compound (29).

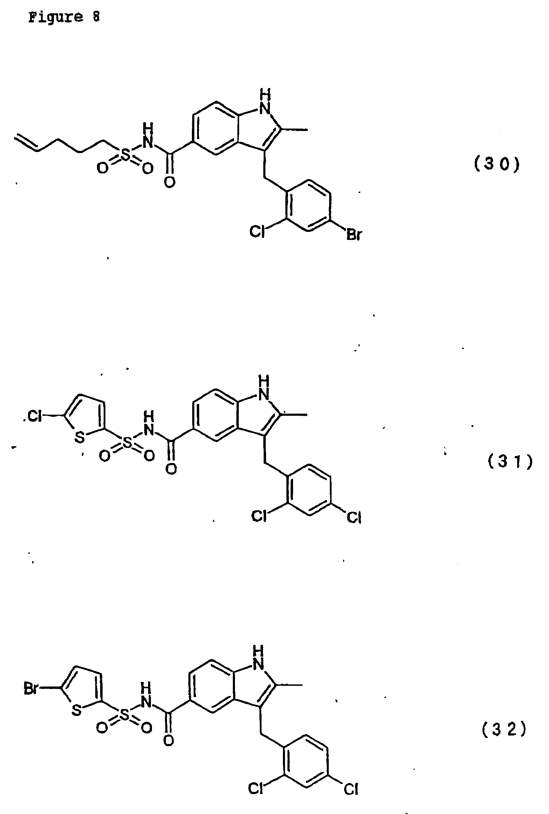

Fig. 8 shows chemical formulae of compound (30) to compound (32).

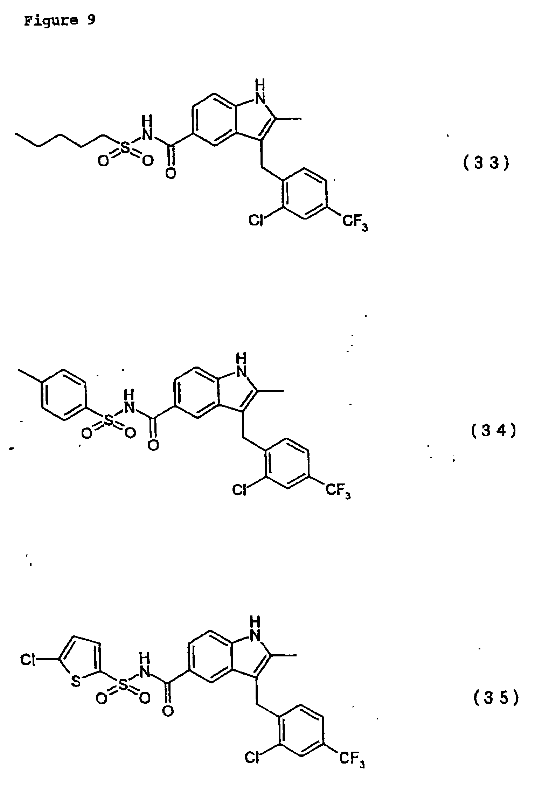

Fig. 9 shows chemical formulae of compound (33) to compound (35).

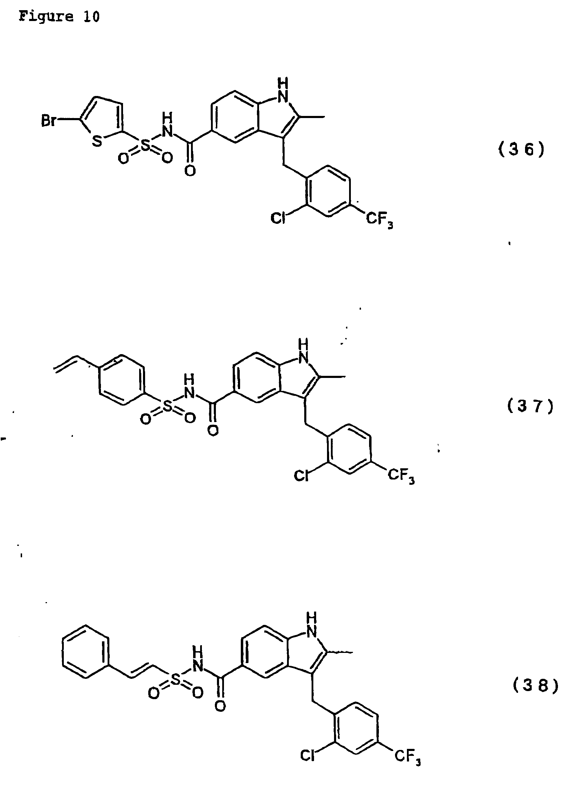

Fig. 10 shows chemical formulae of compound (36) to compound (38).

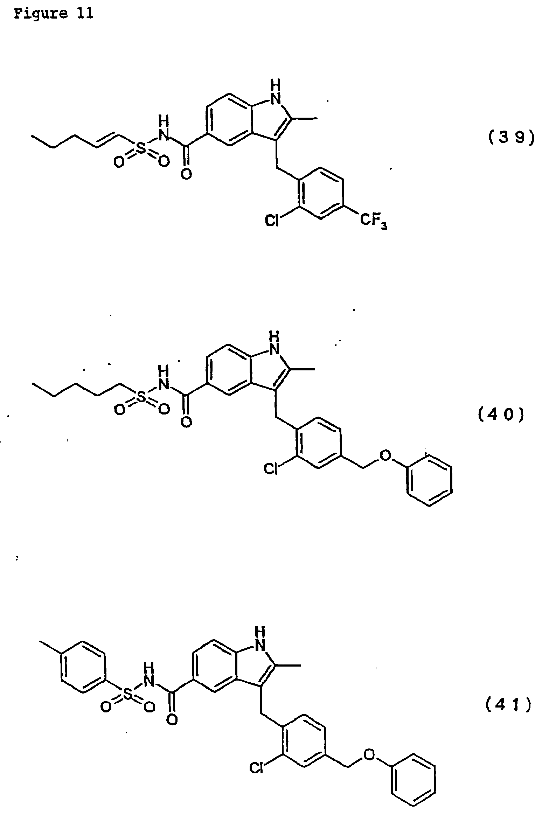

Fig. 11 shows chemical formulae of compound (39) to compound (41).

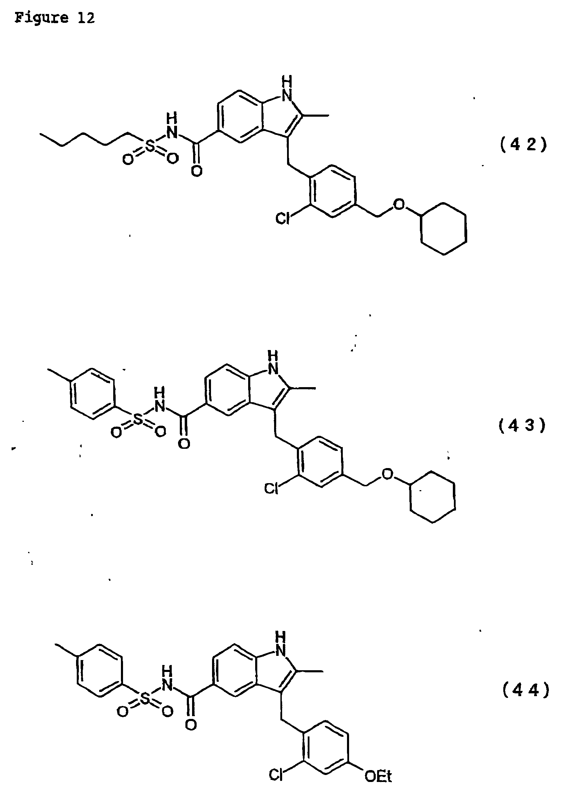

Fig. 12 shows chemical formulae of compound (42) to compound (44).

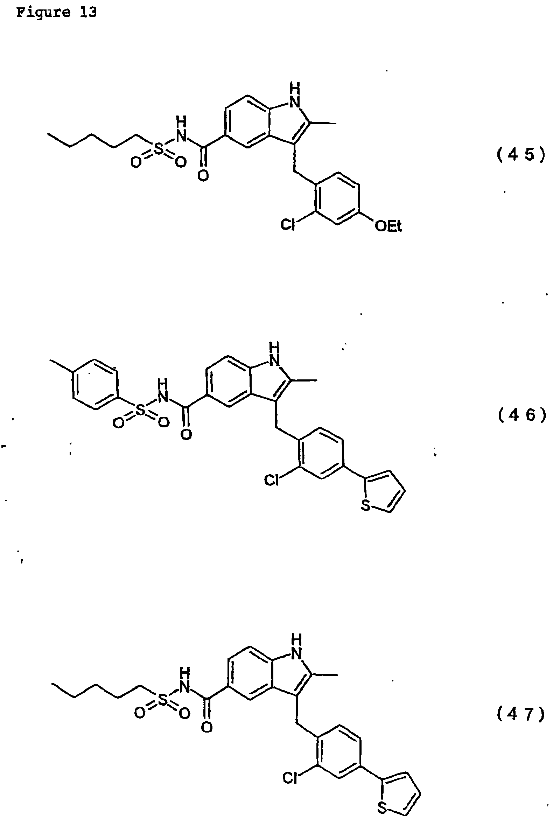

Fig. 13 shows chemical formulae of compound (45) to compound (47).

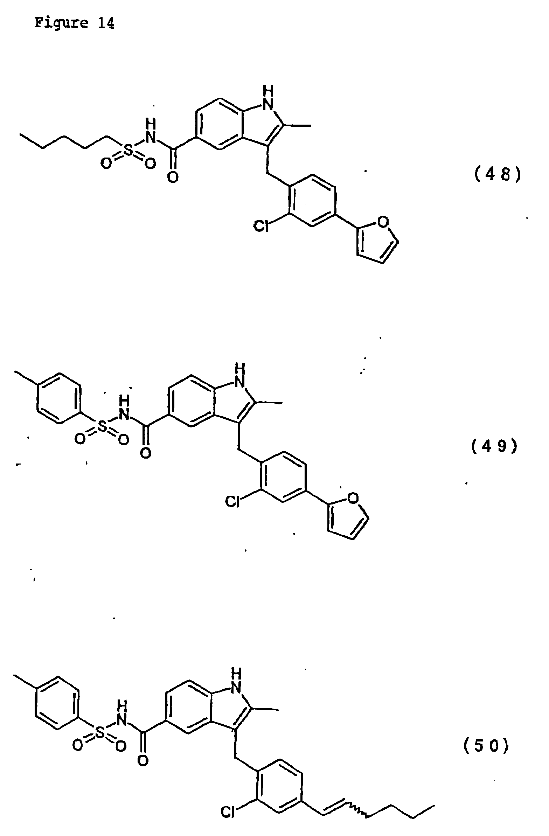

Fig. 14 shows chemical formulae of compound (48) to compound (50).

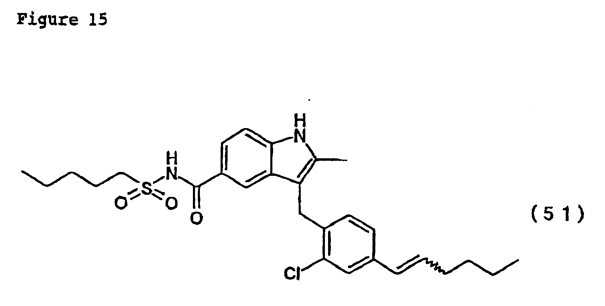

Fig. 15 shows chemical formula of compound (51).

### BEST MODE FOR CARRYING OUT THE INVENTION

The present invention is illustrated more specifically by referring to the Examples below. However, the present invention is not limited thereto.

### Production Example 1

### Production of 3-(2-chloro-4-iodobenzyl)-5-(methoxycarbonyl)-2-methylindole (step 1)

A mixture of 5-(methoxycarbonyl)-2-methylindole (6.62 g), 2-chloro-4-iodobenzyl bromide (32.0 g), L-tartaric acid (12.44 g), sodium hydroxide (3.32 g), 1,4-dioxane (100 ml) and water (55 ml) was stirred at 95°C for 55 hours. The mixture was cooled down to room temperature and then a precipitated solid material was separated by filtration. The solid material was washed with water, with hexane, and then with isopropanol, and dried to give 3-(2-chloro-4-iodobenzyl)-5-(methoxycarbonyl)-2-methylindole (7.27 g).
¹H-NMR (CDCl₃, δ ppm): 2.35(3H, s), 3.89(3H, s), 4.09(2H, s), 6.63(1H, d, J=8.2Hz), 7.30(1H, d, J=8.6Hz), 7.36(1H, d, J=8.2Hz), 7.73(1H, d, J=1.4Hz), 7.85(1H, d, J=8.5Hz), 8.07(1H, brs), 8.08(1H, s)

### Production of 5-carboxy-3-(2-chloro-4-iodobenzyl)-2-methylindole (step 2)

A mixture of 3-(2-chloro-4-iodobenzyl)-5-(methoxycarbonyl)-2-methylindole (1.00 g), a 10% aqueous solution of sodium hydroxide (5 ml), and ethanol (5 ml) was heat-refluxed for 1 hour. The reaction solution was cooled down and then the pH was adjusted to 6 with 1N hydrochloric acid. A precipitated solid material was collected, washed with water and then with a mixed solution of water and ethanol, and dried to yield white crystals of 5-carboxy-3-(2-chloro-4-iodobenzyl)-2-methylindole (0.640 g).
¹H-NMR (DMSO-d₆, δ ppm): 2.32(3H, s), 4.04(2H, s), 6.75(1H, d, J=8.2Hz), 7.30(1H, d, J=8.5Hz), 7.52(1H, d, J=8.1Hz), 7.62(1H, d, J=8.4Hz), 7.80(1H, s), 7.87(1H, s), 11.27(1H, s), 12.28(1H, brs)

### Production Example 2

### Production of 3-(2-chloro-4-phenylethenyl)benzyl)-5-(methoxycarbonyl)-2-methylindole (step 1)

A mixture of 3-(2-chloro-4-iodobenzyl)-5-(methoxycarbonyl)-2-methylindole (0.88 g), phenylacetylene (1.02 g), palladium (II) acetate (0.090 g), triphenylphosphine (0.21 g), tri-n-butylamine (0.75 g), copper (I) iodide (0.12 g) and N,N-dimethylformamide (15 ml) was stirred at 60°C overnight. The solvent was distilled off under reduced pressure, and a mixed solution of ethanol and water was added thereto. The resulting insoluble material was separated by filtration and dried to obtain 3-(2-chloro-4- phenylethenyl)benzyl)-5-(methoxycarbonyl)-2-methylindole (1.00 g).
¹H-NMR (CDCl₃, δ ppm): 2.36(3H, s), 3.89(3H, s), 4.17(2H, s), 6.89(1H, d, J=7.5Hz), 7.21(1H, dd, J=8.0 and 1.7Hz), 7.24-7.53(5H, m), 7.58(1H, d, J=1.7Hz), 7.68-7.71(1H, m), 7.85(1H, dd, J=8.6 and 1.6Hz), 8.07(1H, brs), 8.12(1H, s)

### Production of 5-carboxy-3-(2-chloro-4-phenylethenyl)benzyl)-2-methylindole (step 2)

According to the method used in step 2 of production Example 1, 5-carboxy-3-(2-chloro-4-phenylethenyl)benzyl)-2-methylindole (0.75 g) was obtained from 3-(2-chloro-4-phenylethenyl)benzyl)-5-(methoxycarbonyl)-2-methylindole (1.00 g).
¹H-NMR (DMSO-d₆, δ ppm): 2.34(3H, s), 4.12(2H, s), 7.02(1H, d, J=7.8Hz), 7.20-7.70(1H,m), 7.85-7.95(1H,m), 11.27(1H, s), 12.24(1H, brs)

### Production Example 3

### Production of 3-(2-chloro-4-(2-phenylethenyl)benzyl)-5-(methoxycarbonyl)-2-methylindole (step 1)

A mixture of 3-(2-chloro-4-iodobenzyl)-5-(methoxycarbonyl)-2-methylindole (1.32 g), styrene (1.57 g), palladium (II) acetate (0.090 g), triphenylphosphine (0.21 g), tri-n-butylamine (1.10 g), and N,N-dimethylformamide (25 ml) was stirred at 60°C overnight. The solvent was distilled off under reduced pressure, and a mixed solution of ethanol and water was added thereto. The resulting insoluble material was separated by filtration and dried to obtain 3-(2-chloro-4-(2-phenylethenyl)benzyl)-5-(methoxycarbonyl)-2-methylindole (1.00 g).
¹H-NMR (CDCl₃, δ ppm): 2.35 and 2.38(3H, 2s), 3.88(3H, s), 4.17(2H, s), 6.90-8.17(13H, m)

### Production of 5-carboxy-3-(2-chloro-4-(2-phenylethenyl)benzyl)-2-methylindole (step 2)

According to the method used in step 2 of Production Example 1, 5-carboxy-3-(2-chloro-4-(2-phenylethenyl)benzyl)-2-methylindole (0.83 g) was obtained from 3-(2-chloro-4-(2-phenylethenyl)benzyl)-5-(methoxycarbonyl)-2-methylindole (1.00 g).
¹H-NMR (DMSO-d₆, δ ppm): 2.33 and 2.35(3H, 2s), 4.09(2H, s), 6.98-7.92(13H, m), 11.22(1H, s)

### Production Example 4

### Production of 3-(2-chloro-4-t-butylthiobenzyl)-5-(methoxycarbonyl)-2-methylindole (step 1)

A mixture of 3-(2-chloro-4-iodobenzyl)-5-(methoxycarbonyl)-2-methylindole (0.498 g), tetrakis triphenylphosphine palladium (0) (0.262 g), tri-n-butylamine (0.420 g), t-butylmercaptan (0.510 g), and N,N-dimethylformamide (5 ml) was stirred at 60°C overnight. The solvent was distilled off under reduced pressure, and the obtained residue was purified by silica gel column chromatography (eluate: hexane/ethyl acetate = 2/1) to give 3-(2-chloro-4-(t-butylthio)benzyl)-5-(methoxycarbonyl)-2-methylindole (0.360 g).
¹H-NMR (CDCl₃, δ ppm): 1.55(9H, s), 2.36(3H, s), 3.88(3H, s), 4.16(2H, s), 6.87(1H, d), 7.20-7.33(2H, m), 7.58(1H, s), 7.86(1H, d), 8.06(1H, brs), 8.12(1H, s)

### Production of 5-carboxy-3-(2-chloro-4-(t-butylthio)benzyl)-2-methylindole (step 2)

A mixture of 3-(2-chloro-4-(t-butylthio)benzyl)-5-(methoxycarbonyl)-2-methylindole (0.340 g), a 5% aqueous solution of sodium hydroxide (2.0 g), methanol (2.0 g), ethanol (5 ml), tetrahydrofuran (2 ml), and water (2 ml) was stirred at 80°C for 5 hours. The reaction solution was concentrated to a volume of approximately 1/2 of the original volume and the pH of the solution was adjusted to 3 with 1N hydrochloric acid. Precipitated crystals were collected, washed with water, and dried to give 5-carboxy-3-(2-chloro-4-(t-butylthio)benzyl)-2-methylindole (0.277 g).
¹H-NMR (DMSO-d₆, δ ppm): 1.20(9H, s), 2.33(3H, s), 4.12(2H, s), 7.02(1H, d, J=7.9Hz), 7.30(2H, m), 7.52(1H, s), 7.62(1H, d, J=8.4Hz), 11.27(1H, brs)

### Production Example 5

### Production of 5-carboxy-3-(2-chloro-4-(benzyloxy)benzyl)-2-methylindole (steps 1 and 2)

A mixture of 5-(methoxycarbonyl)-2-methylindole (0.380 g), 2-chloro-4-benzyloxybenzyl chloride (1.068 g), L-tartaric acid (0.750 g), sodium hydroxide (0.200 g), sodium iodide (0.15 g), 1,4-dioxane (6 ml), and water (3 ml) was stirred at 95°C for 46 hours. The reaction solution was concentrated and then subjected to extraction with ethyl acetate, followed by successive washing with water, 1N hydrochloric acid, and a 10% aqueous solution of sodium hydroxide. The separated ethyl-acetate layer was concentrated. Ethanol (7 ml) and a 10% aqueous solution of sodium hydroxide (5 ml) were added to the residual material containing 3-(2-chloro-4-(benzyloxy)benzyl)-5-(methoxycarbonyl)-2-methylindole, and the mixture was heat-refluxed for 1 hour. The reaction solution was cooled down to room temperature and then the pH was adjusted to about 5 with 1N hydrochloric acid. The solution was subjected to extraction with ethyl acetate and washed with water. The separated ethyl-acetate layer was concentrated to yield oily material (0.41 g) containing 5-carboxy-3-(2-chloro-4-(benzyloxy)benzyl)-2-methylindole.
¹H-NMR (DMSO-d₆, δ ppm): 2.32(3H, s), 4.01(2H, s), 5.05(2H, s), 6.84(1H, dd, J=8.6 and 2.6Hz), 7.11(1H, d, J=7.5Hz), 7.27-7.44(6H, m), 7.61(1H, d, J=8.6Hz), 7.89(1H, s), 11.22(1H, s)

### Production Example 6

### Production of 3-(2-chloro-4-(cyclohexylmethyloxy)benzyl)-5-(methoxycarbonyl)-2-methylindole (step 1)

A mixture of 5-(methoxycarbonyl)-2-methylindole (0.170 g), 2-chloro-4-(cyclohexylmethyloxy)benzyl chloride (0.49 g), L-tartaric acid (0.300 g), sodium hydroxide (0.080 g), sodium iodide (0.075 g), 1,4-dioxane (3 ml), and water (1.5 ml) was stirred at 80°C for 40 hours. The reaction solution was concentrated and then subjected to extraction with ethyl acetate, followed by successive washing with water, 1N hydrochloric acid, and a 10% aqueous solution of sodium hydroxide. The separated ethyl-acetate layer was concentrated, and the residual material was washed with water and then with ethanol to obtain white crystals (0.23 g) of 3-(2-chloro-4-(cyclohexylmethyloxy)benzyl)-5-(methoxycarbonyl)-2-methylindole.
¹H-NMR (CDCl₃, δ ppm): 0.97-1.06(2H, m), 1.14-1.33(3H, m), 1.66-1.86(6H, m), 2.36(3H, s), 3.68(2H, d, J=6.4Hz), 3.89(3H, s), 4.09(2H, s), 6.60(1H, dd, J=8.6 and 2.5Hz), 6.81(1H, d, J=8.5Hz), 6.94(1H, d, J=2.5Hz), 7.29(1H, d, J=8.4Hz), 7.84(1H, dd, J=8.4 and 1.4Hz), 8.00(1H, s), 8.14(1H, s)

### Production of 5-carboxy-3-(2-chloro-4-(cyclohexylmethyloxy) benzyl)-2-methylindole (step 2)

Ethanol (10 ml) and a 10% aqueous solution of sodium hydroxide (5 ml) were mixed with 3-(2-chloro-4-(cyclohexylmethyloxy)-benzyl)-5-(methoxycarbonyl)-2-methylindole (0.220 g), and the mixture was heat-refluxed for 1.5 hours. The reaction solution was cooled down to room temperature, the pH was adjusted to about 6 by using 1N hydrochloric acid, and then the resulting precipitate was collected by filtration. The precipitate was washed with water and with 2-propanol and subsequently dried to give white crystals (0.190 g) of 5-carboxy-3-(2-chloro-4-(cyclohexylmethyloxy)benzyl)-2-methylindole.
¹H-NMR (DMSO-d₆, δ ppm): 0.94-1.03(2H, m), 1.09-1.26(3H, m), 1.58-1.78(6H, m), 2.32(3H, s), 3.72(2H, d, J=6.4Hz), 3.99(2H, s), 6.73(1H, dd, J=8.7 and 2.6Hz), 6.85(1H, d, J=8.6Hz), 6.99(1H, d, J=2.6Hz), 7.23(1H, d, J=8.4Hz), 7.61(1H, dd, J=8.4 and 1.5Hz), 7.86(1H, s), 11.12(1H, s)

### Production Example 7

### Production of 3-(2-chloro-4-(trifluoromethyl)benzyl)-5-(methoxycarbonyl)-2-methylindole (step 1)

Trifluoroacetic acid (11.0 g) and triethylsilane (22.4 g) were mixed in a mixed solvent of dichloromethane (10 ml) and acetonitrile (10 ml), and the mixture was cooled with ice. Thereto, a solution, which was prepared by dissolving 5-(methoxycarbonyl)-2-methylindole (6.07 g) and 2-chloro-4-(trifluoromethyl)benzaldehyde (8.04 g) in a mixed solvent of dichloromethane (30 ml) and acetonitrile (30 ml), was added dropwise over a period of 30 minutes. The mixture was stirred at room temperature for 4 hours, and then trifluoroacetic acid (66.0 g) was added thereto. The mixture was further stirred at room temperature for 17 hours. The reaction solution was cooled with ice, and then a 10% aqueous solution of sodium hydroxide (250 ml) was added slowly thereto. The solution was neutralized by adding 1N hydrochloric acid (40 ml) and the resulting solid material was collected by filtration. The filtrate was subjected to extraction with ethyl acetate (100 ml x 2). The extract was combined with the obtained solid material by filtration, and the solid was dissolved. The solution was dried over anhydrous sodium sulfate and concentrated under reduced pressure. Hexane (200 ml) was added to the obtained concentrated oily residue and the mixture was stirred at room temperature. A precipitated solid material was collected by filtration. The material was purified by recrystalslization from a mixed solvent of ethyl acetate (50 ml) and hexane (200 ml) to obtain pale pink crystals (8.83 g) of 3-(2-chloro-4-(trifluoromethyl)-benzyl)-5-(methoxycarbonyl)-2-methylindole.
¹H-NMR (DMSO-d₆, δ ppm): 2.34(3H, s), 3.76(3H, s), 4.19(2H, s), 7.16(1H, d, J=8.1Hz), 7.35(1H, d, J=8.5Hz), 7.56(1H, d, J=8.1Hz), 7.65(1H. d, J=8.5Hz), 7.86(1H. s), 7.90(1H, s), 11.39(1H, s)

### Production of 3-carboxy-5-(2-chloro-4-(trifluoromethyl)benzyl)-2-methylindole (step 2)

According to the method used in step 2 of Production Example 1, 3-carboxy-5-(2-chloro-4-(trifluoromethyl)benzyl)-2-methylindole (4.7 g) was obtained from 3-(2-chloro-4-(trifluoromethyl)benzyl)-5-(methoxycarbonyl)-2-methylindole (5.2 g). ¹H-NMR (DMSO-d₆, δ ppm): 2.34(3H, s), 4.18(2H, s), 7.17(1H, d, J=8.1Hz), 7.32(1H, d, J=8.3Hz), 7.56(1H, d, J=8.1Hz), 7.63(1H, d, J=8.4Hz), 7.85(1H, s), 7.88(1H, s), 11.33(1H, s)

### Production Example 8

### Production of 3-(2-chloro-4-(phenoxymethyl)benzyl)-5-(methoxycarbonyl)-2-methylindole (step 1)

A mixture of 5-(methoxycarbonyl)-2-methylindole (0.568 g), 2-chloro-4-phenoxymethylbenzyl chloride (1.05 g), L-tartaric acid (1.17 g), sodium hydroxide (0.312 g), sodium iodide (0.225 g), 1,4-dioxane (10 ml), and water (5 ml) was stirred at 80°C for two days. After the mixture was cooled down to room temperature, water (50 ml) and ethyl acetate (50 ml) were added thereto for separation. The organic layer was dried over anhydrous sodium sulfate and concentrated under reduced pressure. The concentrated residue obtained was purified by silica gel column chromatography (eluate: methanol/chloroform = 2/98) to give a mixture (1.38 g) containing the compound of interest. The mixture was used in the next step without further purification.

### Production of 5-carboxy-3-(2-chloro-4-(phenoxymethyl)benzyl)-2-methylindole (step 2)

The mixture (0.634 g) containing 3-(2-chloro-4-(phenoxymethyl)benzyl)-5-(methoxycarbonyl)-2-methylindole, which was obtained by the above-mentioned method, was mixed with a 10% aqueous solution of sodium hydroxide (4 ml) and ethanol (20 ml). The resulting mixture was heat-refluxed for 3 hours. After the mixture was cooled down to room temperature, the pH was adjusted to about 5 by adding 1N hydrochloric acid (10 ml). Ethyl acetate (100 ml) heated to 40 to 50°C and water (100 ml) were added thereto for separation. The organic layer was dried over anhydrous sodium sulfate and concentrated under reduced pressure. The obtained residue was purified by silica gel column chromatography (eluate: methanol/chloroform = 5/95) to give 5-carboxy-3-(2-chloro-4-(phenoxymethyl)benzyl)-2-methylindole (0.380 g).
¹H-NMR (DMSO-d₆, δ ppm): 2.35(3H, s), 4.10(2H, s), 5.03(2H, s), 6.93(1H, t, J=7.1Hz), 6.96-7.01(3H, m), 7.23-7.32(4H, m), 7.52(1H, s), 7.62(1H, d, J=8.5Hz), 7.91(1H, s), 11.26(1H, s), 12.26(1H, brs)

### Production Example 9

### Production of 3-(2-chloro-4-(cyclohexyloxymethyl)benzyl)-5-methoxycarbonyl)-2-methylindole (step 1)

A mixture of 5-(methoxycarbonyl)-2-methylindole (0.568 g), 2-chloro-4-(cyclohexyloxymethyl)benzyl chloride (1.09 g), L-tartaric acid (1.17 g), sodium hydroxide (0.312 g), sodium iodide (0.225 g), 1,4-dioxane (10 ml), and water (5 ml) was stirred at 80°C for two days. After the mixture was cooled down to room temperature, water (50 ml) and ethyl acetate (50 ml) were added thereto for separation. The organic layer was dried over anhydrous sodium sulfate and concentrated under reduced pressure. The concentrated residue obtained was purified by silica gel column chromatography (eluate: methanol/chloroform = 2/98) and further purified by recrystalslization from a mixed solvent of ethyl acetate (2 ml) and hexane (6 ml) to give a mixture (0.9 g) containing the compound of interest. The mixture was used in the next step without further purification.

### Production of 5-carboxy-3-(2-chloro-4-(cyclohexyloxymethyl) benzyl)-2-methylindole (step 2)

The mixture (0.9 g) containing 3-(2-chloro-4-(cyclohexyloxymethyl)benzyl)-5-(methoxycarbonyl)-2-methylindole, which was obtained by the above-mentioned method, was mixed with a 10% aqueous solution of sodium hydroxide (4 ml) and ethanol (20 ml). The resulting mixture was heat-refluxed for 3 hours. After the mixture was cooled down to room temperature, the pH was adjusted to about 4 by adding 1N hydrochloric acid (10 ml). Ethyl acetate (100 ml) heated to 40 to 50°C and water (100 ml) were added thereto for separation. The organic layer was dried over anhydrous sodium sulfate and concentrated under reduced pressure. The obtained residue was purified by silica gel column chromatography (eluate: methanol/chloroform = 5/95) to give a mixture (0.57 g) containing 5-carboxy-3-(2-chloro-4-(cyclohexyloxymethyl)benzyl)-2-methylindole. The mixture was used in the next step without further purification.

### Production Example 10

### Production of 3-(2-chloro-4-ethoxybenzyl)-5-(methoxycarbonyl)-2-methylindole (step 1)

Trifluoroacetic acid (0.91 g) and triethylsilane (1.86 g) were mixed in dichloromethane (5 ml), and the mixture was cooled with ice. Thereto, a solution, which was prepared by dissolving 5-(methoxycarbonyl)-2-methylindole (0.50 g) and 2-chloro-4-ethoxybenzaldehyde (0.49 g) in a mixed solvent of dichloromethane (10 ml) and tetrahydrofuran (10 ml), was added dropwise over a period of 10 minutes. The mixture was stirred while being ice-cooled for 10 minutes, and then it was stirred at room temperature for 2 hours. Chloroform (5 ml) and hexane (30 ml) were added to the residue resulted from concentrating the reaction solution. The resulting precipitate was collected by filtration. Dichloromethane (10 ml), trifluoroacetic acid (0.91 g), and triethylsilane (1.86 g) were added to the precipitate, and the mixture was stirred at room temperature for 20 hours. The reaction solution was concentrated, purified by silica gel column chromatography (eluate: ethyl acetate/hexane = 1/3), and further purified by recrystalslization from ethyl acetate/hexane to give 3-(2-chloro-4-ethoxybenzyl)-5-(methoxycarbonyl)-2-methylindole (0.52 g).
¹H-NMR (CDCl₃, δ ppm): 1.37(3H, t, J=6.9Hz), 2.35(3H, s), 3.88(3H, s), 3.97(2H, q, J=7.0Hz), 4.09(2H, s), 6.61(1H, d, J=2.5 and 8.5Hz), 6.82(1H, d, J=8.5Hz), 6.94(1H, d, J=2.5Hz), 7.29(1H, d, J=8.7Hz), 7.83(1H, dd, J=1.5 and 8.5Hz), 8.03(1H, brs), 8.19(1H, s)

### Production of 5-carboxy-3-(2-chloro-4-ethoxybenzyl)-2-methylindole (step 2)

According to the method used in step 2 of Production Example 1, 5-carboxy-3-(2-chloro-4-ethoxybenzyl)-2-methylindole (0.382 g) was obtained from 3-(2-chloro-4-ethoxybenzyl)-5-(methoxycarbonyl)-2-methylindole (0.52 g).
¹H-NMR (DMSO-d₆, δ ppm): 1.27(3H, t, J=6.9Hz), 2.33(3H, s), 3.97(2H, q, J=7.0Hz), 4.01(2H, s), 6.74(1H, dd, J=2.5 and 8.6Hz), 6.88(1H, d, J=8.6Hz), 6.99(1H, d, J=2.5Hz), 7.29(1H, d, J=8.4Hz), 7.61(1H, d, J=8.4Hz), 7.89(1H, s), 11.22(1H, s), 12.25(1H, brs)

### Production Example 11

### Production of 3-(2-chloro-4-(thiophen-2-yl)benzyl)-5-(methoxycarbonyl)-2-methylindole (step 1)

A mixture of 3-(chloro-4-iodobenzyl)-5-(methoxycarbonyl)-2-methylindole (1.00 g), thiophene-2-boric acid (0.35 g), tetrakis triphenylphosphine palladium (0) (0.06 g), ethanol (1 ml), toluene (3 ml), and a 2M sodium carbonate aqueous solution (2.3 ml) was stirred at 90°C for 2 hours. The reaction solution was cooled down to room temperature, and toluene (50 ml) and water (50 ml) were added thereto for separation. The organic layer was filtered through anhydrous sodium sulfate and celite. The residue obtained by concentration under reduced pressure was recrystalslized from ethanol/water (5 ml/5 ml) to yield 3-(2-chloro-4-(thiophen-2-yl)benzyl)-5-(methoxycarbonyl)-2-methylindole (0.95 g).
¹H-NMR (DMSO-d₆, δ ppm): 2.36(3H, s), 3.76(3H, s), 4.11(2H, s), 7.01(1H, d, J=8.1Hz), 7.11(1H, t, J=4.3Hz), 7.34(1H, d, J=8.5Hz), 7.45(1H, d, J=8.1Hz), 7.53(2H, m), 7.64(1H, dd, J=1.3 and 8.5Hz), 7.73(1H, d, J=1.5Hz), 7.94(1H, s), 11.34(1H, s)

### Production of 5-carboxy-3-(2-chloro-4-(thiophen-2-yl)benzyl)-2-methylindole (step 2)

According to the method used in step 2 of Production Example 1, 5-carboxy-3-(2-chloro-4-(thiophen-2-yl)benzyl)-2-methylindole (0.28 g) was obtained from 3-(2-chloro-4-(thiophen-2-yl)benzyl)-5-(methoxycarbonyl)-2-methylindole (0.95 g).
¹H-NMR (DMSO-d₆, δ ppm): 2.36(3H, s), 4.11(2H, s), 7.02(1H, d, J=8.2Hz), 7.11(1H, m), 7.31(1H, d, J=8.4Hz), 7.45(1H, dd, J=1.6 and 8.0Hz), 7.53(2H, m), 7.63(1H, dd, J=1.3 and 8.4Hz), 7.73(1H, d, J=1.5Hz), 7.93(1H, s), 11.27(1H, s), 12.26(1H, brs)

### Production Example 12

### Production of 3-(2-chloro-4-(furan-2-yl)benzyl)-5-(methoxycarbonyl)-2-methylindole (step 1)

A mixture of 3-(chloro-4-iodobenzyl)-5-(methoxycarbonyl)-2-methylindole (1.00 g), furan-2-boric acid (0.34 g), tetrakis triphenylphosphine palladium (O) (0.06 g), ethanol (1 ml), toluene (3 ml) and a 2M sodium carbonate aqueous solution (2.5 ml) was stirred at 90°C for 2.5 hours. The reaction solution was cooled down to room temperature, and toluene (50 ml) and water (50 ml) were added thereto for separation. The organic layer was filtered through celite. The resultant solution was dried over anhydrous sodium sulfate and then concentrated under reduced pressure. The obtained residue was recrystalslized from ethanol/water (20 ml/20 ml) to yield 3-(2-chloro-4-(thiophen-2-yl)benzyl)-5-(methoxycarbonyl)-2-methylindole (0.57 g).
¹H-NMR (DMSO-d₆, δ ppm): 2.35(3H, s), 3.76(3H, s), 4.11(2H, s), 5.57(1H, dd, J=3.3 and 1.8Hz), 6.98(1H, d, J=3.3Hz), 7.04(1H, d, J=8.2Hz), 7.34(1H, d, J=8.5Hz), 7.49(1H, d, J=8.1Hz), 7.64(1H, d, J=8.5Hz), 7.73(1H, s), 7.76(1H, d, J=1.4Hz), 7.93(1H, s), 11.33(1H, s)

### Production of 5-carboxy-3-(2-chloro-4-(furan-2-yl)benzyl)-2-methylindole (step 2)

According to the method used in step 2 of Production Example 1, 5-carboxy-3-(2-chloro-4-(furan-2-yl)benzyl)-2-methylindole (0.51 g) was obtained from 3-(2-chloro-4-(furan-2-yl)benzyl)-5-(methoxycarbonyl)-2-methylindole (0.57 g).
¹H-NMR (DMSO-d₆, δ ppm): 2.36(3H, s), 4.11(2H, s), 6.57(1H, d, J=2.5Hz), 6.97(1H, d, J=3.1Hz), 7.05(1H, d, J=8.1Hz), 7.31(1H, d, J=8.5Hz), 7.49(1H, d, J=8.2Hz), 7.63(1H, d, J=8.4Hz), 7.72(1H, s), 7.76(1H, s), 7.92(1H, s), 11.26(1H, s), 12.26(1H, brs)

### Production Example 13

### Production of 3-(2-chloro-4-(1-hexen-1-yl)benzyl-5-(methoxycarbonyl)-2-methylindole (step 1)

A mixture of 3-(2-chloro-4-iodobenzyl)-5-(methoxycarbonyl)-2-methylindole (0.88 g), 1-hexene (0.84 g), palladium (II) acetate (0.068 g), triphenylphosphine (0.160 g), tri-n-butylamine (1.12 g), and N,N-dimethylformamide (15 ml) was stirred at 60°C for 5 hours. The reaction solution was concentrated under reduced pressure, and ethanol (10 ml) was added to the residue. An insoluble material was removed by filtration, and water (100 ml) and ethyl acetate (100 ml) were added to the solution for separation. The organic layer was dried over anhydrous sodium sulfate and concentrated under reduced pressure. The obtained residue was purified by silica gel column chromatography (eluate: ethyl acetate/hexane = 1/3) to give a mixture (0.29 g) of 3-(2-chloro-4-(1-hexen-1-yl)benzyl)-5-(methoxycarbonyl)-2-methylindole and 3-(2-chloro-4-(1-hexen-2-yl)benzyl)-5-(methoxycarbonyl)-2-methylindole. The mixture was used in the next step without further purification.
mp: 141-146°C

### Production of 5-carboxy-3-(2-chloro-4-(1-hexen-1-yl)benzyl)-2-methylindole (step 2)

According to the method used in step 2 of Production Example 1, a mixture (0.22 g) of 5-carboxy-3-(2-chloro-4-(1-hexen-1-yl)benzyl)-2-methylindole and 5-carboxy-3-(2-chloro-4-(1-hexen-2-yl)benzyl)-2-methylindole was obtained from a mixture (0.29 g) of 3-(2-chloro-4-(1-hexen-1-yl)benzyl)-5-methoxycarbonyl)-2-methylindole and 3-(2-chloro-4-(1-hexen-2-yl)benzyl)-5-(methoxycarbonyl)-2-methylindole. The mixture was used in the next step without further purification.

### Example 1

### Synthesis of 3-(2-chloro-4-(t-butylthio)benzyl)-2-methyl-5-(1-pentanesulfonylcarbamoyl)indole (compound (9))

N,N'-carbonyldiimidazole (0.108 g) was added to a mixture of 5-carboxy-3-(2-chloro-4-(t-butylthio)benzyl)-2-methylindole (0.152 g) and N,N-dimethylformamide (2 ml), and then the resulting mixture was stirred at room temperature for 40 minutes. Subsequently, thereto, an N,N-dimethylformamide solution (2 ml) containing 1-pentanesulfonamide (0.095 g) and diazabicycloundecene (0.090 g) was added, and the mixture was stirred at 100°C overnight. The solvent was distilled off under reduced pressure. Methanol and water were added to the residue, and the pH of the solution was adjusted to 3 by adding 1N hydrochloric acid thereto. The mixture was extracted twice with ethyl acetate. The organic layer was dried, concentrated, and then purified by preparative thin layer chromatography (developing solvent: ethyl acetate/hexane = 1/1). Further, the material was recrystalslized from a mixed solvent of methanol and water to obtain white crystals (0.103 g) of 3-(2-chloro-4-(t-butylthio)benzyl)-2-methyl-5-(1-pentanesulfonylcarbamoyl)indole.
¹H-NMR (DMSO-d₆, δ ppm): 0.80(3H, t, J=7.3Hz), 1.20-1.38(13H, m), 1.66(2H, m), 2.29(3H, s), 3.47(2H, m), 4.13(2H, s), 6.96(1H, d, J=8.0Hz), 7.30(1H, d, J=7.9Hz), 7.35(1H, d, J=8.5Hz), 7.53(1H, s), 7.63(1H, d, J=8.5Hz), 8.05(1H, s), 11.38(1H, s), 11.67(1H, s)
mp: 185-187.5°C

### Example 2

### Synthesis of 3-(2-chloro-4-(t-butylthio)benzyl)-2-methyl-5-(4-methylbenzene)sulfonylcarbamoyl)indole (compound (10))

According to the method used in Example 1, a foamy solid material (0.155 g) of 5-((4-methylbenzene)sulfonylcarbamoyl)-3-(2-chloro-4-(t-butylthio)benzyl)-2-methylindole was obtained from 5-carboxy-3-(2-chloro-4-t-butylthiobenzyl)-2-methylindole (0.120 g), N,N'-carbonyldiimidazole (0.085 g), (4-methylbenzene)-sulfonamide (0.079 g), and diazabicycloundecene (0.071 g).
¹H-NMR (CDCl₃, δ ppm): 1.24(9H, s), 2.28(3H, s), 2.37(3H, s), 4.04(2H, s), 6.73(1H, d, J=7.9Hz), 7.12(1H, d, J=7.9Hz), 7.23-7.31(3H, m), 7.48-7.52(2H, m), 7.87(1H, s), 7.99(2H, d, J=8.3Hz), 8.47(1H, brs) IR (Nujol): 1682 cm⁻¹

### Example 3

### Synthesis of 3-(2-chloro-4-iodobenzyl)-2-methyl-5-(1-pentanesulfonylcarbamoyl)indole (compound (11))

According to the method used in Example 1, 3-(2-chloro-4-iodobenzyl)-2-methyl-5-(1-pentanesulfonylcarbamoyl)indole (0.350 g) was obtained from 5-carboxy-3-(2-chloro-4-iodobenzyl)-2-methylindole (0.30 g), N,N'-carbonyldiimidazole (0.23 g), 1-pentanesulfonamide (0.22 g), and diazabicycloundecene (0.22 ml).
¹H-NMR (DMSO-d₆, δ ppm): 0.81(3H, t, J=7.1Hz), 1.22-1.39(4H, m), 1.63-1.71(2H, m), 2.29(3H, s), 3.47(2H, t, J=7.4Hz), 4.05(2H, s), 6.69(1H, d, J=8.1Hz), 7.34(1H, d, J=8.3Hz), 7.52(1H, d, J=8.2Hz), 7.62(1H, d, J=8.6Hz), 7.81(1H, s), 8.02(1H, s), 11.37(1H, s), 11.69(1H, s)
mp: 188-189°C

### Example 4

### Synthesis of 3-(2-chloro-4-iodobenzyl)-2-methyl-5-((4-methylbenzene)sulfonylcarbamoyl)indole (compound (12))

According to the method used in Example 1, 3-(2-chloro-4-iodobenzyl)-2-methyl-5-((4-methylbenzene)sulfonylcarbamoyl)-indole (0.350 g) was obtained from 5-carboxy-3-(2-chloro-4-iodobenzyl)-2-methylindole (0.30 g), N,N'-carbonyldiimidazole (0.23 g), (4-methylbenzene)sulfonamide (0.24 g), and diazabicycloundecene (0.22 ml).
¹H-NMR (DMSO-d₆, δ ppm): 2.27(3H, s), 2.37(3H, s), 4.03(2H, s), 6.67(1H, d, J=8.1Hz), 7.30(1H, d, J=8.5Hz), 7.40(2H, d, J=8.1Hz), 7.51(1H, d, J=7.7Hz), 7.53(1H, d, J=8.2Hz), 7.81(1H, s), 7.85(2H, d, J=8.0Hz), 7.95(1H, s), 11.34(1H, s), 12.12(1H, brs)
mp: 283-285°C

### Example 5

### Synthesis of 3-(2-chloro-4-(phenylethynyl)benzyl)-2-methyl-5-(1-pentanesulfonylcarbamoyl)indole (compound (13))

According to the method used in Example 1, 3-(2-chloro-4-(phenylethynyl)benzyl)-2-methyl-5-(1-pentanesulfonylcarbamoyl)-indole (0.050 g) was obtained from 5-carboxy-3-(2-chloro-4-(phenylethynyl)benzyl)-2-methylindole (0.28 g), N,N'-carbonyldiimidazole (0.23 g), 1-pentanesulfonamide (0.21 g), and diazabicycloundecene (0.21 ml).
¹H-NMR (DMSO-d₆, δ ppm): 0.80(3H, t, J=7.3Hz), 1.21-1.38(4H, m), 1.63-1.70(2H, m), 2.31(3H, s), 3.47(2H, t, J=7.7Hz), 4.14(2H, s), 6.98(1H, d, J=8.0Hz), 7.34-7.38(2H, m), 7.40-7.43(3H, m), 7.52-7.55(2H, m), 7.63(1H, d, J=8.5Hz), 7.66(1H, s), 8.05(1H, s), 11.39(1H, s), 11.68(1H, s)
mp: 206-207°C

### Example 6

### Synthesis of 3-(2-chloro-4-(phenylethynyl)benzyl)-2-methyl-5-((4-methylbenzene)sulfonylcarbamoyl)indole (compound (14))

According to the method used in Example 1, 3-(2-chloro-4-(phenylethynyl)benzyl)-2-methyl-5-((4-methylbenzene)sulfonylcarbamoyl)indole (0.020 g) was obtained from 5-carboxy-3-((2-chloro-4-phenylethynyl)benzyl)-2-methylindole (0.28 g), N,N'-carbonyldiimidazole (0.23 g), (4-methylbenzene)sulfonamide (0.24 g), and diazabicycloundecene (0.21 ml).
¹H-NMR (DMSO-d₆, δ ppm): 2.29(3H, s), 2.36(3H, s), 4.12(2H, s), 6.95(1H, d, J=8.1Hz), 7.30(1H, d, J=8.4Hz), 7.34-7.44(6H, m), 7.52-7.56(3H, m), 7.66(1H, s), 7.84(2H, d, J=7.7Hz), 7.97(1H, s), 11.35(1H, s), 12.09(1H, s)
mp: 203-205°C

### Example 7

### Synthesis of 3-(2-chloro-4-(2-phenylethenyl)benzyl)-2-methyl-5-((4-methylbenzene)sulfonylcarbamoyl)indole (compound (15))

According to the method used in Example 1, white crystals (0.184 g) of 3-(2-chloro-4-(2-phenylethenyl)benzyl)-2-methyl-5-((4-methylbenzene)sulfonylcarbamoyl)indole were obtained from 5-carboxy-3-(2-chloro-4-(2-phenylethenyl)benzyl)-2-methylindole (0.399 g), N,N'-carbonyldiimidazole (0.242 g), (4-methylbenzene)sulfonamide (0.255 g), and diazabicycloundecene (0.227 g).
¹H-NMR (DMSO-d₆, δ ppm): 2.37(3H, s), 2.45(3H, s), 4.10(2H, s), 6.95(1H, d, J=8.2Hz), 7.18-7.32(3H, m), 7.34-7.41(6H, m), 7.53(1H, d), 7.57(2H, d, J=7.3Hz), 7.71(1H, s), 7.84(2H, d, J=8.3Hz), 8.00(1H, s), 11.34(1H, s), 12.10(1H, s)
mp: 207-208.5°C

### Example 8

### Synthesis of 3-(2-chloro-4-(2-phenylethenyl)benzyl)-2-methyl-5-(1-pentanesulfonylcarbamoyl)indole (compound (16))

According to the method used in Example 1, white crystals (0.038 g) of 3-(2-chloro-4-(2-phenylethenyl)benzyl)-2-methyl-5-(1-pentanesulfonylcarbamoyl)indole were obtained from 5-carboxy-3-(2-chloro-4-(2-phenylethenyl)benzyl)-2-methylindole (0.150 g), N,N'-carbonyldiimidazole (0.091 g), 1-pentanesulfonamide (0.085 g), and diazabicycloundecene (0.085 g).
¹H-NMR (DMSO-d₆, δ ppm): 0.79(3H, t, J=7.3Hz), 1.25(2H, m), 1.34(2H, m), 1.67(2H, m), 2.32(3H, s), 3.46(2H, m), 6.97(1H, d, J=8.2Hz), 7.16-7.29(3H, m), 7.33-7.42(4H, m), 7.56(2H, d, J=7.8Hz), 7.63(1H, d), 7.71(1H, s), 8.07(1H, s), 11.36(1H, s), 11.69(1H, s)
mp: 205.5-207°C

### Example 9

### Synthesis of 3-(2-chloro-4-(2-phenylethyl)benzyl)-2-methyl-5-((4-methylbenzene)sulfonylcarbamoyl)indole (compound (17))

In an atmosphere of nitrogen, platinum dioxide (0.010 g) was added to a mixture of 3-(2-chloro-4-(2-phenylethenyl)benzyl)-2-methyl-5-((4-methylbenzene)sulfonylcarbamoyl)indole (0.098 g) obtained in Example 7, acetic acid (4 ml), and ethyl acetate (10 ml). The mixture was hydrogenated and stirred at room temperature for 90 minutes. The resulting solid material was removed by filtration and the filtrate was concentrated. The obtained residue was recrystalslized from a mixed solvent of methanol and water to give white solid material (0.068 g) of 3-(2-chloro-4-(2-phenylethyl)benzyl)-2-methyl-5-((4-methylbenzene)sulfonylcarbamoyl)indole.
¹H-NMR (DMSO-d₆, δ ppm): 2.27(3H, s), 2.36(3H, s), 2.81(4H, s), 4.04(2H, s), 6.83(1H, d, J=8.0Hz), 7.00-7.32(8H, m), 7.40(2H, d, J=7.3Hz), 7.53(1H, d, J=8.3Hz), 7.85(2H, d, J=8.2Hz), 7.97(1H, s), 11.31(1H, s), 12.09(1H, s) Mass(FAB⁺): m/e 557(M+1)
mp: 207-208°C

### Example 10

### Synthesis of 3-(2-chloro-4-(benzyloxy)benzyl)-2-methyl-5-((4-methylbenzene)sulfonylcarbamoyl)indole (compound (18))

According to the method used in Example 1, pale yellow crystals (0.120 g) of 3-(2-chloro-4-(benzyloxy)benzyl)-2-methyl-5-((4-methylbenzene)sulfonylcarbamoyl)indole were obtained from 5-carboxy-3-(2-chloro-4-(benzyloxy)benzyl)-2-methylindole (0.400 g), N,N'-carbonyldiimidazole (0.320 g), (4-methylbenzene)sulfonamide (0.330 g), and diazabicycloundecene (0.300 g).
¹H-NMR (DMSO-d₆, δ ppm): 2.28(3H, s), 2.36(3H, s), 4.00(2H, s), 5.06(2H, s), 6.82(2H, d, J=1.4Hz), 7.11(1H, s), 7.27-7.42(9H, m), 7.52(1H, dd, J=8.6 and 1.7Hz), 7.84(1H, d, J=8.3Hz), 7.96(1H, s), 11.29(1H, s), 12.10(1H, brs)
mp: 173-174°C

### Example 11

### Synthesis of 3-(2-chloro-4-(cyclohexylmethyloxy)benzyl)-2-methyl-5-((4-methylbenzene)sulfonylcarbamoyl)indole (compound (19))

According to the method used in Example 1, white crystals (0.180 g) of 3-(2-chloro-4-(cyclohexylmethyloxy)benzyl)-2-methyl-5-((4-methylbenzene)sulfonylcarbamoyl)indole were obtained from 5-carboxy-3-(2-chloro-4-(cyclohexylmethyloxy)benzyl)-2-methylindole (0.180 g), N,N'-carbonyldiimidazole (0.200 g), (4-methylbenzene)sulfonamide (0.220 g), and diazabicycloundecene (0.190 g).
¹H-NMR (DMSO-d₆, δ ppm): 0.94-1.03(2H, m), 1.09-1.27(3H, m), 1.58-1.78(6H, m), 2.27(3H, s), 2.37(3H, s), 3.72(2H, d, J=6.4Hz), 3.99(2H, s), 6.73(1H, dd, J=8.6 and 2.6Hz), 6.80(1H, d, J=8.7Hz), 7.00(1H, d, J=2.5Hz), 7.28(1H, d, J=8.6Hz), 7.39(2H, d, J=8.0Hz), 7.52(1H, d, J=8.5Hz), 7.84(2H, d, J=8.2Hz), 7.96(1H, s), 11.28(1H, s), 12.10(1H, brs)
mp: 167-168°C
IR (Nujol): 1683cm⁻¹

### Example 12

### Synthesis of 3-(2-chloro-4-phenylbenzyl)-5-((5-chloro-2-thiophenesulfonyl)carbamoyl)-2-methylindole (compound (20))

According to the method used in Example 1, pale yellow powder (0.170 g) of 3-(2-chloro-4-phenylbenzyl)-5-((5-chloro-2-thiophenesulfonyl)carbamoyl)-2-methylindole was obtained from 5-carboxy-3-(2-chloro-4-phenylbenzy)-2-methylindole (0.200 g), N,N'-carbonyldiimidazole (0.130 g), 5-chlorothiophene-2-sulfonamide (0.130 g), and diazabicycloundecene (0.120 g).
¹H-NMR (DMSO-d₆, δ ppm): 2.32(3H, s), 4.13(2H, s), 6.97(1H, d, J=8.1Hz), 7.12-7.64(10H, m), 7.73(1H, d, J=1.9Hz), 8.00 (1H, s), 11.30(1H, brs), 12.50(1H, brs)
mp: 200-201°C
IR (Nujol): 1678cm⁻¹

### Example 13

### Synthesis of 3-(2-chloro-4-phenylbenzyl)-5-((5-bromo-2-thiophenesulfonyl)carbamoyl)-2-methylindole (compound (21))

According to the method used in Example 1, pale yellow crystals (0.390 g) of 5-((5-bromo-2-thiophenesulfonyl)carbamoyl)-3-(2-chloro-4-phenylbenzyl)-2-methylindole were obtained from 5-carboxy-3-(2-chloro-4-phenylbenzy)-2-methylindole (0.270 g), N,N'-carbonyldiimidazole (0.170 g), (5-bromothiophen-2-yl)-sulfonamide (0.250 g), and diazabicycloundecene (0.160 g).
¹H-NMR (DMSO-d₆, δ ppm): 2.33(3H, s), 4.14 (2H, s), 6.98 (1H, d, J=8.1Hz), 7.33-7.37(3H, m), 7.41-7.48(3H, m), 7.58-7.65(4H, m), 7.74(1H, d, J=1.8Hz), 8.05(1H, s), 11.40(1H, s), 12.50(1H, brs)
mp: 198-200°C
IR (Nujol): 1674cm⁻¹

### Example 14

### Synthesis of 3-(2-chloro-4-phenylbenzyl)-2-methyl-5-(4-pentenesulfonylcarbamoyl)indole (compound (22))

According to the method used in Example 1, crystals (0.105 g) of 3-(4-bromo-2-chlorobenzyl)-2-methyl-5-(4-pentenesulfonylcarbamoyl)indole was obtained from 5-carboxy-3-(2-chloro-4-phenylbenzy)-2-methylindole (0.200 g), N,N'-carbonyldiimidazole (0.172 g), 4-pentenesulfonamide (0.159 g), and diazabicycloundecene (0.162 g).
¹H-NMR (DMSO-d₆, δ ppm): 1.72-1.80(2H, m), 2.09-2.15(2H, m), 2.34(3H, s), 3.47(2H, t, J=7.8Hz), 4.15(2H, s), 4.94(1H, d, J=9.9Hz), 4.99(1H, d, J=17.1Hz), 5.68-5.79(1H, m), 7.00(1H, d, J=8.0Hz), 7.37(2H, m), 7.39-7.50(3H, m), 7.63(3H, m), 7.74(1H, s), 8.09(1H, m), 11.39(1H, s), 11.73(1H, brs)
mp: 131-137°C

### Example 15

### Synthesis of 3-((1-bromonaphthalen-2-yl)methyl)-5-((5-chloro-2-thiophenesulfonyl)carbamoyl)-2-methylindole (compound (23))

According to the method used in Example 1, pale brown powder (0.180 g) of 3-((1-bromonaphthalen-2-yl)methyl)-5-((5-chloro-2-thiophenesulfonyl)carbamoyl)-2-methylindole were obtained from 3-((1-bromonaphthalen-2-yl)methyl)-5-carboxy-2-methylindole (0.210 g), N,N'-carbonyldiimidazole (0.130 g), 5-chloro-2-thiophenesulfonamide (0.130 g), and diazabicycloundecene (0.120 g).
¹H-NMR (DMSO-d₆, δ ppm): 2.31(3H, s), 4.36(2H, s), 7.10(1H, d, J=8.6Hz), 7.23(1H, d, J=4.1Hz), 7.34(1H, d, J=8.6Hz), 7.53-7.60(2H, m), 7.65-7.69(2H, m), 7.78(1H, d, J=8.5Hz), 7.89(1H, d, J=8.1Hz), 8.05 (1H, s), 8.26(1H, d, J=8.6Hz), 11.40(1H, brs), 12.50(1H, brs)
mp: 216-218°C
IR (Nujol): 1672cm⁻¹

### Example 16

### Synthesis of 3-((1-bromonaphthalen-2-yl)methyl)-5-((5-bromo-2-thiophenesulfonyl)carbamoyl)-2-methylindole (compound (24))

According to the method used in Example 1, pale yellow crystals (0.230 g) of 3-((1-bromonaphthalen-2-yl)methyl)-5-((5-bromo-2-thiophenesulfonyl)carbamoyl)-2-methylindole were obtained from 3-((1-bromonaphthalen-2-yl)methyl)-5-carboxy-2-methylindole (0.220 g), N,N'-carbonyldiimidazole (0.150 g), 5-bromo-2-thiophenesulfonamide (0.220 g), and diazabicycloundecene (0.140 g).
¹H-NMR (DMSO-d₆, δ ppm): 2.31(3H, s), 4.37(2H, s), 7.10(1H, d, J=8.5Hz), 7.32-7.36(2H, m), 7.55(1H, t, J=7.4Hz), 7.59(1H, d, J=8.6Hz), 7.63(1H, d, J=4.0Hz), 7.67(1H, t, J=7.7Hz), 7.78(1H, d, J=8.5Hz), 7.89(1H, d, J=8.1Hz), 8.07(1H, s), 8.27(1H, d, J=8.6Hz), 11.41(1H, brs), 12.47(1H, brs)
mp: 225.5-226.5°C
IR (Nujol): 1674cm⁻¹

### Example 17

### Synthesis of 3-(4-bromo-2-chlorobenzyl)-2-methyl-5-((4-methylbenzene)sulfonylcarbamoyl)indole (compound (25))

According to the method used in Example 1, pale red powder (0.440 g) of 3-(4-bromo-2-chlorobenzyl)-2-methyl-5-((4-methylbenzene)sulfonylcarbamoyl)indole was obtained from 3-(4-bromo-2-chlorobenzyl)-5-carboxy-2-methylindole (0.390 g), N,N'-carbonyldiimidazole (0.290 g), (4-methylbenzene)sulfonamide (0.300 g), and diazabicycloundecene (0.270 g).
¹H-NMR (DMSO-d₆, δ ppm): 2.27(3H, s), 2.36(3H, s), 4.04(2H, s), 6.84(1H, d, J=8.3Hz), 7.28(1H, d, J=8.6Hz), 7.35-7.40(3H, m), 7.54(1H, d, J=8.7Hz), 7.71(1H, d, J=1.9Hz), 7.83(2H, d, J=8.2Hz), 7.94 (1H, s), 11.31(1H, s), 12.10(1H, brs)
mp: 226-228°C
IR (Nujol): 1682cm⁻¹

### Example 18

### Synthesis of 3-(4-bromo-2-chlorobenzyl)-2-methyl-5-((4-vinylbenzene)sulfonylcarbamoyl)indole (compound (26))

According to the method used in Example 1, white crystals (0.190 g) of 3-(4-bromo-2-chlorobenzyl)-2-methyl-5-((4-vinylbenzene)-sulfonylcarbamoyl)indole were obtained from 3-(4-bromo-2-chloro-benzyl)-5-carboxy-2-methylindole (0.390 g), N,N'-carbonyl-diimidazole (0.290 g), (4-vinylbenzene)sulfonamide (0.320 g), and diazabicycloundecene (0.270 g).
¹H-NMR (DMSO-d₆, δ ppm): 2.28(3H, s), 4.05(2H, s), 5.46(1H, d, J=10.9Hz), 6.01(1H, d, J=17.7Hz), 6.78-6.86(2H, m), 7.31(1H, d, J=8.5Hz), 7.37(1H, dd, J=8.4 and 1.6Hz), 7.54(1H, d, J=8.4Hz), 7.69(2H, d, J=8.4Hz), 7.71(1H, d, J=1.9Hz), 7.92(2H, d, J=8.3Hz), 7.97 (1H, s), 11.37(1H, s), 12.16(1H, brs)
mp: 215°C (decomp.)
IR (Nujol): 1679cm⁻¹

### Example 19

### Synthesis of 3-(4-bromo-2-chlorobenzyl)-2-methyl-5-((2-phenylethenyl)sulfonylcarbamoyl)indole (compound (27))

According to the method used in Example 1, pale red crystals (0.300 g) of 3-(4-bromo-2-chlorobenzyl)-2-methyl-5-((2-phenylethenyl)sulfonylcarbamoyl)indole were obtained from 3-(4-bromo-2-chlorobenzyl)-5-carboxy-2-methylindole (0.390 g), N,N'-carbonyldiimidazole (0.290 g), (2-phenylethenyl)sulfonamide (0.320 g), and diazabicycloundecene (0.270 g).
¹H-NMR (DMSO-d₆, δ ppm): 2.28(3H, s), 4.05(2H, s), 6.83(1H, d, J=8.4Hz), 7.35(1H, d, J=8.7Hz), 7.37(1H, dd, J=8.3 and 2.0Hz), 7.41-7.47(3H, m), 7.48(1H, d, J=15.4Hz), 7.58-7.64(2H, m), 7.71(1H, d, J=2.0Hz), 7.73-7.76(2H, m), 8.04(1H, s), 11.37(1H, s), 11.86(1H, brs)
mp: 204.5-205.5°C
IR (Nujol): 1674cm⁻¹

### Example 20

### Synthesis of 3-(4-bromo-2-chlorobenzyl)-2-methyl-5-((1-pentene)-sulfonylcarbamoyl)indole (compound (28))

According to the method used in Example 1, pale yellow crystals (0.050 g) of 3-(4-bromo-2-chlorobenzyl)-2-methyl-5-((1-pentene)-sulfonylcarbamoyl)indole were obtained from 3-(4-bromo-2-chlorobenzyl)-5-carboxy-2-methylindole (0.390 g), N,N'-carbonyldiimidazole (0.290 g), (1-pentene)sulfonamide (0.270 g), and diazabicycloundecene (0.270 g).
¹H-NMR (DMSO-d₆, δ ppm): 0.86(3H, t, J=7.4Hz), 1.40-1.47(2H, m), 2.21(2H, quartet, J=6.6Hz), 2.29(3H, s), 4.05(2H, s), 6.76(1H, s), 6.84(1H, d, J=8.3Hz), 7.32(1H, d, J=8.5Hz), 7.37(1H, d, J=8.3Hz), 7.41-7.51(1H, m), 7.60(1H, d, J=8.4Hz), 7.71(1H, d, J=1.9Hz), 7.99(1H, s), 11.34(1H, s), 11.73(1H, brs)
mp: 163-164°C
IR (Nujol): 1680cm⁻¹

### Example 21

### Synthesis of 3-(4-bromo-2-chlorobenzyl)-5-((5-bromo-2-thiophenesulfonyl)carbamoyl)-2-methylindole (compound (29))

According to the method used in Example 1, pale red crystals (0.230 g) of 3-(4-bromo-2-chlorobenzyl)-5-((5-bromo-2-thiophenesulfonyl)carbamoyl)-2-methylindole were obtained from 3-(4-bromo-2-chlorobenzyl)-5-carboxy-2-methylindole (0.270 g), N,N'-carbonyldiimidazole (0.170 g), 5-bromo-2-thiophenesulfonamide (0.250 g), and diazabicycloundecene (0.160 g).
¹H-NMR (DMSO-d₆, δ ppm): 2.28(3H, s), 4.06(2H, s), 6.84 (1H, d, J=8.4Hz), 7.34(1H, d, J=8.7Hz), 7.35(1H, d, J=4.1Hz), 7.38(1H, dd, J=8.4 and 2.0Hz), 7.59(1H, dd, J=8.6 and 1.7Hz), 7.65(1H, d, J=4.1Hz), 7.71(1H, d, J=2.0Hz), 7.99(1H, s), 11.41(1H, s), 12.50(1H, brs)
mp: 234-235°C
IR (Nujol): 1689cm⁻¹

### Example 22

### Synthesis of 3-(4-bromo-2-chlorobenzyl)-2-methyl-5-(4-pentenesulfonylcarbamoyl)indole (compound (30))

According to the method used in Example 1, crystals (0.032 g) of 3-(4-bromo-2-chlorobenzyl)-2-methyl-5-(4-pentenesulfonylcarbamoyl)indole was obtained from 3-(4-bromo-2-chlorobenzyl)-5-carboxy-2-methylindole (0.200 g), N,N'-carbonyldiimidazole (0.171 g), 4-pentenesulfonamide (0.160 g), and diazabicycloundecene (0.158 g).
¹H-NMR(DMSO-d₆, δ ppm): 1.73-1.81(2H, m), 2.11-2.16(2H, m), 2.30(3H, s), 3.47(2H, m), 4.06(2H, s), 4.99(2H, m), 5.70-5.99(1H, m), 6.86(1H, d, J=8.4Hz), 7.34(1H, d, J=8.5Hz), 7.38(1H, d, J=8.2Hz), 7.63(1H, d, J=8.3Hz), 7.72(1H, s), 8.03(1H, s), 11.38(1H, brs), 11.71(1H, brs)
mp: 145-150°C

### Example 23

### Synthesis of 5-((5-chloro-2-thiophenesulfonyl)carbamoyl)-3-(2,4-dichlorobenzyl)-2-methylindole (compound (31))

According to the method used in Example 1, pale yellow crystals (0.450 g) of 5-((5-chloro-2-thiophenesulfonyl)carbamoyl)-3-(2,4-dichlorobenzyl)-2-methylindole were obtained from 5-carboxy-3-(2,4-dichlorobenzyl)-2-methylindole (0.330 g), N,N'-carbonyldiimidazole (0.240 g), 5-chloro-2-thiophenesulfonamide (0.300 g), and diazabicycloundecene (0.230 g).
¹H-NMR (DMSO-d₆, δ ppm): 2.29(3H, s), 4.07(2H, s), 6.91(1H, d, J=8.4Hz), 7.23-7.27(2H, m), 7.34(1H, d, J=8.5Hz), 7.58-7.61(2H, m), 7.69(1H, d, J=4.1Hz), 7.99(1H, s), 11.40(1H, s), 12.48 (1H, brs)
mp: 212-214°C
IR (Nujol): 1688cm⁻¹

### Example 24

### Synthesis of 5-((5-bromo-2-thiophenesulfonyl)carbamoyl)-3-(2,4-dichlorobenzyl)-2-methylindole (compound (32))

According to the method used in Example 1, pale yellow crystals (0.460 g) of 5-((5-bromo-2-thiophenesulfonyl)carbamoyl)-3-(2,4-dichlorobenzyl)-2-methylindole were obtained from 5-carboxy-3-(2,4-dichlorobenzyl)-2-methylindole (0.330 g), N,N'-carbonyldiimidazole (0.240 g), 5-bromo-2-thiophenesulfonamide (0.360 g), and diazabicycloundecene (0.230 g).
¹H-NMR (DMSO-d₆, δ ppm): 2.28(3H, s), 4.07(2H, s), 6.91(1H, d, J=8.4Hz), 7.25(1H, dd, J=8.4 and 2.2Hz), 7.34(1H, d, J=8.5Hz), 7.36(1H, d, J=4.0Hz), 7.59(1H, dd, J=8.6 and 1.6Hz), 7.61(1H, d, J=2.1Hz), 7.65(1H, d, J=4.0Hz), 8.00(1H, s), 11.41(1H, s), 12.48 (1H, brs)
mp: 231-233°C
IR (Nujol): 1688cm⁻¹

### Example 25

### Synthesis of 3-(2-chloro-4-(trifluoromethyl)benzyl)-2-methyl-5-(1-pentanesulfonylcarbamoyl)indole (compound (33))

According to the method used in Example 1, white crystals (0.225 g) of 3-(2-chloro-4-(trifluoromethyl)benzyl)-2-methyl-5-(1-pentanesulfonylcarbamoyl)indole were obtained from 5-carboxy-3-(2-chloro-4-(trifluoromethyl)benzyl)-2-methylindole (0.200 g), N,N'-carbonyldiimidazole (0.177 g), 1-pentanesulfonamide (0.166 g), and diazabicycloundecene (0.166 g).
¹H-NMR (DMSO-d₆, δ ppm): 0.79(3H, t, J=7.2Hz), 1.25(2H, m), 1.34(2H, m), 1.66(2H, m), 2.31(3H, s), 3.47(2H, t, J=7.6Hz), 4.18(2H, s), 7.11(1H, d, J=8.1Hz), 7.36(1H, d, J=8.5Hz), 7.55(1H, d, J=8.1Hz), 7.63(1H, d, J=8.5Hz), 7.86(1H, s), 8.04(1H, s), 11.43(1H, s), 11.92(1H, brs)
mp: 146-150°C

### Example 26

### Synthesis of 3-(2-chloro-4-(trifluoromethyl)benzyl)-2-methyl-5-(4-methylbenzenesulfonylcarbamoyl)indole (compound (34))

According to the method used in Example 1, white crystals (0.220 g) of 3-(2-chloro-4-(trifluoromethyl)benzyl)-2-methyl-5-(4-methylbenzenesulfonylcarbamoyl)indole were obtained from 5-carboxy-3-(2-chloro-4-(trifluoromethyl)benzyl)-2-methylindole (0.200 g), N,N'-carbonyldiimidazole (0.177 g), p-toluenesulfonamide (0.187 g), and diazabicycloundecene (0.166 g).
¹H-NMR (DMSO-d₆, δ ppm): 2.29(3H, s), 2.37(3H, s), 4.17(2H, s), 7.09(1H, d, J=8.1Hz), 7.32(1H, d, J=8.5Hz), 7.39(2H, d, J=8.2Hz), 7.55(2H, d, J=8.5Hz), 7.84(3H, m), 7.98(1H, s), 11.41(1H, s), 12.12(1H, brs)
mp: 247-250°C

### Example 27

### Synthesis of 3-(2-chloro-4-(trifluoromethyl)benzyl)-2-methyl-5-((5-chloro-2-thiophenesulfonyl)carbamoyl)indole (compound (35))

According to the method used in Example 1, white crystals (0.295 g) of 3-(2-chloro-4-(trifluoromethyl)benzyl)-2-methyl-5-((5-chloro-2-thiophenesulfonyl)carbamoyl)indole were obtained from 5-carboxy-3-(2-chloro-4-(trifluoromethyl)benzyl)-2-methylindole (0.368 g), N,N'-carbonyldiimidazole (0.243 g), 5-chloro-2-thiophenesulfonamide (0.297 g), and diazabicycloundecene (0.228 g).
¹H-NMR (DMSO-d₆, δ ppm): 2.30(3H, s), 4.18(2H, s), 7.09(1H, d, J=8.0Hz), 7.25(1H, d, J=4.0Hz), 7.35(1H, d, J=8.5Hz), 7.55(1H, d, J=8.2Hz), 7.60(1H, d, J=8.8Hz), 7.69(1H, d, J=4.0Hz), 7.86(1H, s), 8.00(1H, s), 11.44(1H, s), 12.51(1H, brs)
IR: 1696cm⁻¹
mp: 228-230°C

### Example 28

### Synthesis of 3-(2-chloro-4-(trifluoromethyl)benzyl)-2-methyl-5-((5-bromo-2-thiophenesulfonyl)carbamoyl)indole (compound (36))

According to the method used in Example 1, white crystals (0.425 g) of 3-(2-chloro-4-(trifluoromethyl)benzyl)-2-methyl-5-((5-bromo-2-thiophenesulfonyl)carbamoyl)indole were obtained from 5-carboxy-3-(2-chloro-4-(trifluoromethyl)benzyl)-2-methylindole (0.368 g), N,N'-carbonyldiimidazole (0.243 g), 5-bromo-2-thiophenesulfonamide (0.363 g), and diazabicycloundecene (0.228 g).
¹H-NMR (DMSO-d₆, δ ppm): 2.30(3H, s), 4.18(2H, s), 7.09(1H, d, J=8.1Hz), 7.35(2H, m), 7.55(1H, d, J=8.2Hz), 7.60(1H, dd, J=1.6 and 8.6Hz), 7.64(1H, d, J=4.1Hz), 7.86(1H, s), 8.01(1H, s), 11.44(1H, s), 12.45(1H, brs)
IR: 1691cm⁻¹
mp: 247-249°C

### Example 29

### Synthesis of 3-(2-chloro-4-(trifluoromethyl)benzyl)-2-methyl-5-((4-vinylbenzene)sulfonylcarbamoyl)indole (compound (37))

According to the method used in Example 1, pale yellowish brown crystals (0.420 g) of 3-(2-chloro-4-(trifluoromethyl)benzyl)-2-methyl-5-((4-vinylbenzene)sulfonylcarbamoyl)indole were obtained from 5-carboxy-3-(2-chloro-4-(trifluoromethyl)benzyl)-2-methylindole (0.368 g), N,N'-carbonyldiimidazole (0.243 g), (4-vinylbenzene)sulfonamide (0.275 g), and diazabicycloundecene (0.228 g).
¹H-NMR (DMSO-d₆, δ ppm): 2.29(3H, s), 4.17(2H, s), 5.45(1H, d, J=11.0Hz), 6.00(1H, d, J=17.6Hz), 6.81(1H, dd, J=17.6 and 11.0Hz), 7.09(1H, d, J=8.1Hz), 7.32(1H, d, J=8.5Hz), 7.55(2H, m), 7.68(2H, d, J=8.4Hz), 7.86(1H, s), 7.92(2H, d, J=8.4Hz), 7.98(1H, s), 11.40(1H, s), 12.15(1H, brs)
IR: 1681cm⁻¹
mp: 185-188°C

### Example 30

### Synthesis of 3-(2-chloro-4-(trifluoromethyl)benzyl)-2-methyl-5-((2-phenylethenyl)sulfonylcarbamoyl)indole (compound (38))

According to the method used in Example 1, pale yellowish brown crystals (0.215 g) of 3-(2-chloro-4-(trifluoromethyl)benzyl)-2-methyl-5-((2-phenylethenyl)sulfonylcarbamoyl)indole was obtained from 5-carboxy-3-(2-chloro-4-(trifluoromethyl)benzyl)-2- methylindole (0.368 g), N,N'-carbonyldiimidazole (0.243 g), (2-phenylethenyl)sulfonamide (0.275 g), and diazabicycloundecene (0.228 g).
¹H-NMR (DMSO-d₆, δ ppm): 2.30(3H, s), 4.18(2H, s), 7.09(1H, d, J=8.0Hz), 7.35(1H, d, J=8.5Hz), 7.44(3H, m), 7.48(1H, d, J=15.6Hz), 7.55(1H, d, J=8.0Hz), 7.61(1H, d, J=15.8Hz), 7.63(1H, m), 7.75(2H, d, J=6.5Hz), 7.876(1H, s), 8.06(1H, s), 11.41(1H, s), 11.96(1H, brs)
IR: 1688cm⁻¹
mp: 219-224°C

### Example 31

### Synthesis of 3-(2-chloro-4-(trifluoromethyl)benzyl)-2-methyl-5-((1-pentene)sulfonylcarbamoyl)indole (compound (39))

According to the method used in Example 1, crystals (0.105 g) of 3-(2-chloro-4-(trifluoromethyl)benzyl)-2-methyl-5-((1-pentene)sulfonylcarbamoyl)indole were obtained from 5-carboxy-3-(2-chloro-4-(trifluoromethyl)benzyl)-2-methylindole (0.368 g), N,N'-carbonyldiimidazole (0.243 g), 1-pentenesulfonamide (0.224 g), and diazabicycloundecene (0.228 g).
¹H-NMR (DMSO-d₆, δ ppm): 0.85(3H, t, J=7.4Hz), 1.43(2H, m), 2.22(2H, g, J=7.0Hz), 2.30(3H, s), 4.18(2H, s), 6.75(1H, d, J=15.2Hz), 6.82(1H, m), 7.09(1H, d, J=8.1Hz), 7.35(1H, d, J=8.5Hz), 7.55(1H, d, J=8.0Hz), 7.61(1H, d, J=7.3Hz), 7.86(1H, s), 8.02(1H, s), 11.41(1H, s), 11.76(1H,brs)
IR: 1674cm⁻¹
mp: 90-93°C

### Example 32

### Synthesis of 3-(2-chloro-4-(phenoxymethyl)benzyl)-2-methyl-5-(1-pentanesulfonylcarbamoyl)indole (compound (40))

According to the method used in Example 1, white crystals (0.094 g) of 3-(2-chloro-4-(phenoxymethyl)benzyl)-2-methyl-5-(1-pentanesulfonylcarbamoyl)indole were obtained from 5-carboxy-3-(2-chloro-4-(phenoxymethyl)benzyl)-2-methylindole (0.179 g), N,N'-carbonyldiimidazole (0.143 g), 1-pentanesulfonamide (0.134 g), and diazabicycloundecene (0.133 g).
¹H-NMR (DMSO-d₆, δ ppm): 0.80(3H, t, J=7.2Hz), 1.26(2H, m), 1.34(2H, m), 1.67(2H, m), 2.31(3H, s), 3.47(2H, t, J=7.7Hz), 4.11(2H, s), 5.04(2H, s), 6.90-6.98(4H, m), 7.26(3H, m), 7.34(1H, d, J=8.6Hz), 7.53(1H, s), 7.62(1H, d, J=8.9Hz), 8.05(1H, s), 11.36(1H, s), 11.68(1H, s)
mp: 151-153°C

### Example 33

### Synthesis of 3-(2-chloro-4-(phenoxymethyl)benzyl)-2-methyl-5-(4-methylbenzenesulfonylcarbamoyl)indole (compound (41))

According to the method used in Example 1, pale yellow crystals (0.132 g) of 3-(2-chloro-4-(phenoxymethyl)benzyl)-2-methyl-5-(4-methylbenzenesulfonylcarbamoyl)indole were obtained from 5-carboxy-3-(2-chloro-4-(phenoxymethyl)benzyl)-2-methylindole (0.179 g), N,N'-carbonyldiimidazole (0.143g), p-toluenesulfonamide (0.151 g), and diazabicycloundecene (0.133 g).
¹H-NMR (DMSO-d₆, δ ppm): 2.89(3H, s), 2.36(3H, s), 4.09(2H, s), 5.04(2H, s), 6.91-6.98(4H, m), 7.22-7.31(4H, m), 7.39(2H, d, J=8.2Hz), 7.53(2H, m), 7.85(2H, d, J=8.2Hz), 7.99(1H, s), 11.34(1H, s), 12.09(1H, brs)
mp: 170-172°C

### Example 34

### Synthesis of 3-(2-chloro-4-(cyclohexyloxymethyl)benzyl)-2-methyl-5-(1-pentanesulfonylcarbamoyl)indole (compound (42))

According to the method used in Example 1, pale yellow oily material (0.155 g) of 3-(2-chloro-4-(cyclohexyloxymethyl)-benzyl)-2-methyl-5-(1-pentanesulfonylcarbamoyl)indole was obtained from 5-carboxy-3-(2-chloro-4-(cyclohexyloxymethyl)benzyl)-2- methylindole (0.280 g), N,N'-carbonyldiimidazole (0.220 g), 1-pentanesulfonamide (0.205 g), and diazabicycloundecene (0.205 g).
¹H-NMR (DMSO-d₆, δ ppm): 0.81(3H, t, J=7.1Hz), 1.13-1.40(9H, m), 1.45(1H, m), 1.65(4H, m), 1.83(2H, m), 2.30(3H, s), 3.47(2H, t, J=7.6Hz), 4.09(2H, s), 4.42(2H, s), 4.53(1H, m), 6.92(1H, d, J=7.9Hz), 7.10(1H, d, J=7.9Hz), 7.34(1H, d, J=8.6Hz), 7.38(1H, s), 7.63(1H, d, J=8.5Hz), 8.05(1H, s), 11.34(1H, s), 11.68(1H, brs)

### Example 35

### Synthesis of 3-(2-chloro-4-(cyclohexyloxymethyl)benzyl)-2-methyl-5-(4-methylbenzenesulfonylcarbamoyl)indole (compound (43))

According to the method used in Example 1, pale yellow crystals (0.140 g) of 3-(2-chloro-4-(cyclohexyloxymethyl)benzyl)-2-methyl-5-(4-methylbenzenesulfonylcarbamoyl)indole were obtained from 5-carboxy-3-(2-chloro-4-(cyclohexyloxymethyl)benzyl)-2-methylindole (0.280 g), N,N'-carbonyldiimidazole (0.220 g), p-toluenesulfonamide (0.233 g), and diazabicycloundecene (0.205 g).
¹H-NMR (DMSO-d₆, δ ppm): 1.15-1.30(5H, m), 1.46(1H, m), 1.64(2H, m), 1.83(2H, m), 2.28(3H, s), 2.37(3H, s), 4.07(2H, s), 4.42(2H, s), 5.53(1H, m), 6.89(1H, d, J=8.0Hz), 7.09(1H, d, J=8.0Hz), 7.30(1H, d, J=8.6Hz), 7.37(1H, s), 7.40(2H, d, J=8.1Hz), 7.53(1H, d, J=8.6Hz), 7.85(2H, d, J=8.3Hz), 7.98(1H, s), 11.32(1H, s), 12.09(1H, s)
mp: 178.8-180.9°C

### Example 36

### Synthesis of 3-(2-chloro-4-ethoxybenzyl)-2-methyl-5-(4-methylbenzenesulfonylcarbamoyl)indole (compound (44))

According to the method used in Example 1, colorless crystals (0.145 g) of 3-(2-chloro-4-ethoxybenzyl)-2-methyl-5-(4-methylbenzenesulfonylcarbamoyl)indole were obtained from 5-carboxy-3-(2-chloro-4-ethoxybenzyl)-2-methylindole (0.190 g), N,N'-carbonyldiimidazole (0.162 g), p-toluenesulfonamide (0.171 g), and diazabicycloundecene (0.152 g).
¹H-NMR (DMSO-d₆, δ ppm): 1.27(3H, t, J=7.0Hz), 2.28(3H, s), 2.37(3H, s), 3.97(2H, g, J=7.0Hz), 4.00(2H, s), 6.73(1H, dd, J=8.6 and 2.5Hz), 6.82(1H, d, J=8.6Hz), 7.00(1H, d, J=2.5Hz), 7.29(1H, d, J=8.6Hz), 7.40(2H, d, J=8.2Hz), 7.52(1H, dd, J=8.5 and 1.7Hz), 7.85(2H, d, J=8.3Hz), 7.97(1H, s), 11.30(1H, s), 12.09(1H, s)
mp: 161.9-163.3°C

### Example 37

### Synthesis of 3-(2-chloro-4-ethoxybenzyl)-2-methyl-5-(1-pentanesulfonylcarbamoyl)indole (compound (45))

According to the method used in Example 1, colorless crystals (0.090 g) of 3-(2-chloro-4-ethoxybenzyl)-2-methyl-5-(1-pentanesulfonylcarbamoyl)indole were obtained from 5-carboxy-3-(2-chloro-4-ethoxybenzyl)-2-methylindole (0.190 g), N,N'-carbonyldiimidazole (0.162 g), 1-pentanesulfonamide (0.151 g), and diazabicycloundecene (0.152 g).
¹H-NMR (DMSO-d₆, δ ppm): 0.81(3H, t, J=7.3Hz), 1.27(5H, m), 1.35(2H, m), 1.67(2H, m), 2.29(3H, s), 3.47(2H, t, J=7.7Hz), 3.97(2H, q, J=6.9Hz), 4.02(2H, s), 6.74(1H, dd, J=8.6 and 2.0Hz), 6.84(1H, d, J=8.6Hz), 7.00(1H, d, J=2.0Hz), 7.33(1H, d, J=8.5Hz), 7.61(1H, d, J=8.5Hz), 8.04(1H, s), 11.32(1H, s), 11.68(1H, s)
mp: 103.0-105.5°C

### Example 38

### Synthesis of 3-(2-chloro-4-(thiophen-2-yl)benzyl)-2-methyl-5-(4-methylbenzenesulfonylcarbamoyl)indole (compound (46))

According to the method used in Example 1, colorless crystals (0.045 g) of 3-(2-chloro-4-(thiophen-2-yl)benzyl)-2-methyl-5-(4-methylbenzenesulfonylcarbamoyl)indole were obtained from 5-carboxy-3-(2-chloro-4-(thiophen-2-yl)benzyl)-2-methylindole (0.115 g), N,N'-carbonyldiimidazole (0.073 g), p-toluenesulfonamide (0.077 g), and diazabicycloundecene (0.069 g).
¹H-NMR (DMSO-d₆, δ ppm): 2.30(3H, s), 2.35(3H, s), 4.10(2H, s), 6.95(1H, d, J=8.1Hz), 7.12(1H, dd, J=3.7 and 5.0Hz), 7.30(1H. d, J=8.5Hz), 7.37(2H, d, J=8.2Hz), 7.44(1H, dd, J=1.8 and 8.1Hz), 7.51-7,56(3H, m), 7.73(1H, d, J=1.9Hz), 7.84(2H, d, J=8.3Hz), 8.00(1H, s), 11.34(1H, s), 12.12(1H, brs)
mp: 236.5-242.0°C

### Example 39

### Synthesis of 3-(2-chloro-4-(thiophen-2-yl)benzyl)-2-methyl-5-(1-pentanesulfonylcarbamoyl)indole (compound (47))

According to the method used in Example 1, colorless crystals (0.067 g) of 3-(2-chloro-4-(thiophen-2-yl)benzyl)-2-methyl-5-(1-pentanesulfonylcarbamoyl)indole were obtained from 5-carboxy-3-(2-chloro-4-(thiophen-2-yl)benzyl)-2-methylindole (0.160 g), N,N'-carbonyldiimidazole (0.102 g), 1-pentanesulfonamide (0.095 g), and diazabicycloundecene (0.096 g).
¹H-NMR (DMSO-d₆, δ ppm): 0.79(3H, t, J=7.3Hz), 1.24(2H, m), 1.33(2H, m), 1.66(2H, m), 2.32(3H, s), 3.46(2H, t, J=7.7Hz), 4.12(2H, s), 6.97(1H, d, J=8.1Hz), 7.11(1H, dd, J=4.0 and 4.9Hz), 7.35(1H, d, J=8.5Hz), 7.44(1H, dd, J=1.8 and 8.0Hz), 7.52(1H, d, J=3.2Hz), 7.54(1H, d, J=5.1Hz), 7.63(1H, dd, J=1.5 and 8.5Hz), 7.73(1H, d, J=1.8Hz), 8.07(1H, s), 11.37(1H, s), 11.69(1H, brs)
mp: 184.4-185.1°C

### Example 40

### Synthesis of 3-(2-chloro-4-(furan-2-yl)benzyl)-2-methyl-5-(1-pentanesulfonylcarbamoyl)indole (compound (48))

According to the method used in Example 1, white crystals (0.170 g) of 3-(2-chloro-4-(furan-2-yl)benzyl)-2-methyl-5-(1-pentane-sulfonylcarbamoyl)indole was obtained from 5-carboxy-3-(2-chloro-4-(furan-2-yl)benzyl)-2-methylindole (0.250 g), N,N'-carbonyldiimidazole (0.162 g), 1-pentanesulfonamide (0.151 g), and diazabicycloundecene (0.152 g).
¹H-NMR (DMSO-d₆, δ ppm): 0.79(3H, t, J=7.3Hz), 1.24(2H, m), 1.33(2H, m), 1.65(2H, m), 2.32(3H, s), 3.45(2H, t, J=7.6Hz), 4.12(2H, s), 6.57(1H,m), 6.97(1H, d, J=3.2Hz), 7.00(1H, d, J=8.1Hz), 7.34(1H, d, J=8.5Hz), 7.49(1H, d, J=8.1Hz), 7.62(1H, d, J=8.6Hz), 7.72(1H, s), 7.76(1H, s), 8.06(1H, s), 11.35(1H, s), 11.70(1H, brs)
mp: 162.1-163.8°C
IR: 1652cm⁻¹

### Example 41

### Synthesis of 3-(2-chloro-4-(furan-2-yl)benzyl)-2-methyl-5-(4-methylbenzenesulfonylcarbamoyl)indole (compound (49))

According to the method used in Example 1, white crystals (0.260 g) of 3-(2-chloro-4-(furan-2-yl)benzyl)-2-methyl-5-(4-methylbenzenesulfonylcarbamoyl)indole were obtained from 5-carboxy-3-(2-chloro-4-(furan-2-yl)benzyl)-2-methylindole (0.250 g), N,N'-carbonyldiimidazole (0.162 g), p-toluenesulfonamide (0.171 g), and diazabicycloundecene (0.152 g).
¹H-NMR (DMSO-d₆, δ ppm): 2.30(3H, s), 2.35(3H, s), 4.10(2H, s), 6.58(1H, m), 6.98(2H, m), 7.30(1H, d, J=8.6Hz), 7.38(2H, d, J=8.1Hz), 7.49(1H, d, J=7.9Hz), 7.53(1H, d, J=8.4Hz), 7.73(1H, s), 7.77(1H, s), 7.84(2H, d, J=8.1Hz), 8.00(1H, s), 11.34(1H,s), 12.12(1H, brs)
mp: 232.7-234.1°C
IR: 1679cm⁻¹

### Example 42

### Synthesis of 3-(2-chloro-4-(1-hexen-2-yl)benzyl)-2-methyl-5-(4-methylbenzenesulfonylcarbamoyl)indole and 3-(2-chloro-4-(1-hexen-1-yl)benzyl)-2-methyl-5-(4-methylbenzenesulfonylcarbamoyl)indole (compound (50))

According to the method used in Example 1, pale yellow crystals (0.067 g) of a mixture containing, at an abundance ratio of about 2:8, of 3-(2-chloro-4-(1-hexen-2-yl)benzyl)-2-methyl-5-(4-methylbenzenesulfonylcarbamoyl)indole and 3-(2-chloro-4-(1-hexen-1-yl)benzyl)-2-methyl-5-(4-methylbenzenesulfonylcarbamoyl)indole were obtained from 5-carboxy-3-(2-chloro-4-(1-hexen-1-yl)benzyl)-2-methylindole (0.100 g) containing 5-carboxy-3-(2-chloro-4-(1-hexen-2-yl)benzyl)-2-methylindole, N,N'-carbonyldiimidazole (0.064 g), p-toluenesulfonamide (0.067 g), and diazabicycloundecene (0.060 g).
¹H-NMR(DMSO-d₆, δ ppm): 0.87(3H, m), 1.28-1.61(4H, m), 1.91-2.14(2H, m), 2.28(3H, s), 2.37(3H, s), 4.08(2H, m), 5.05-5.48(1H, m), 5.80/6.30(1H,m), 6.80-7.00(1H, m), 7.17-7.26(1H, m), 7.29(1H, d, J=8.3Hz), 7.39(2H, d, J=7.5Hz), 7.42-7.48(1H, m), 7.53(1H, d, J=8.2Hz), 7.85(2H, d, J=7.8Hz), 7.98(1H, s), 11.31(1H, s), 12.10(1H, brs)
mp: 173-183°C
IR: 1659cm⁻¹

### Example 43

### Synthesis of 3-(2-chloro-4-(1-hexen-2-yl)benzyl-2-methyl-5-(1-pentanesulfonylcarbamoyl)indole and 3-(2-chloro-4-(1-hexen-1-yl)benzyl-2-methyl-5-(1-pentanesulfonylcarbamoyl)indole (compound (51))

According to the method used in Example 1, pale yellow crystals (0.062 g) of a mixture containing, at an abundance ratio of about 2:8, of 3-(2-chloro-4-(1-hexen-2-yl)benzyl)-2-methyl-5-(1-pentanesulfonylcarbamoyl)indole and 3-(2-chloro-4-(1-hexen-1-yl)benzyl)-2-methyl-5-(1-pentanesulfonylcarbamoyl)indole were obtained from 5-carboxy-3-(2-chloro-4-(1-hexen-1-yl)benzyl)-2-methylindole (0.100 g) containing 5-carboxy-3-(2-chloro-4-(1-hexen-2-yl)benzyl)-2-methylindole, N,N'-carbonyldiimidazole (0.064 g), 1-pentanesulfonamide (0.060 g), and diazabicycloundecene (0.060 g).
¹H-NMR(DMSO-d₆, δ ppm): 0.78-0.91(6H, m), 1.20-1.61(8H, m), 1.66(2H, m), 1.91-2.45(2H, m), 2.30(3H, m), 3.47(2H, t, J=7.6Hz), 4.07(2H, m), 5.05-5.82(1H, m), 6.28-6.99(2H, m), 7.16-7.29(1H, m), 7.34(1H, d, J=8.4Hz), 7.42-7.63(2H, m), 8.05(1H, m), 11.33(1H, s), 11.68(1H, s)
mp: 84-85°C
IR: 1666cm⁻¹

### Test Example: Test for activity of decreasing plasma glucose using db/db mice

### Test compounds

3-(1-bromonaphthalen-2-ylmethyl)-5-((5-chloro-2-thiophenylsulfonyl)carbamoyl)-2-methylindole (compound (23))

### Animal used

Five-week-old female mice [C57BL/KsJ-dbm db+/db+, C57BL/KsJ-dbm +m/+m (Jackson Laboratory)] were purchased, and were kept for 2 to 3 weeks. Then, these mice were used in the test.

### Preparation of an agent

A test compound was mixed with a powdered chow (CE-2, made by Nippon Clea) using a mortar. The mixing ratio was 0.01%. The mixed chow was changed twice a week for each group. The feed amount and the remaining amount were recorded, and the intake was calculated from the difference therebetween.

### Test schedule

The female db/db mice were grouped according to the body weight, the plasma glucose, and the plasma triglyceride concentrations. Then, the mixture containing the test compound was administered to the mice for 14 days (from 8 to 10 weeks old). In the morning on day 7 and day 14, the blood was collected from the orbital venous plexus using heparinized glass capillary tubes (Chase Heparinized Capillary Tubes), and a plasma fraction was obtained through centrifugal separation. Plasma glucose, triglyceride, and insulin concentrations were measured on day 0 and day 14 as well as plasma glucose and triglyceride concentrations on day 7. The body weight was measured on day 0, day 7, and day 14. After the final collection of the blood, the mice was killed using CO₂ gas.

### Measurement method

The plasma glucose was measured by a glucose oxidase method (Glucose CII-Test Wako made by Wako Pure Chemical Industries, Ltd.) using from 10 to 15 µl of plasma. The plasma triglyceride concentration was measured by a GPO-p-chlorophenol method (Triglyceride G-Test Wako made by Wako Pure Chemical Industries, Ltd.) or a GPO-DAOS method (Triglyceride E-Test Wako) using from 10 to 15 µl of plasma. The above-mentioned measurements were conducted immediately after the blood collection. The plasma insulin concentration was measured by radio immuno assay method (Phadesef Insulin RIA Kit made by Cabi Pharmacia) using 20 µl of plasma (which can be stored at -20°C).

### Results

The difference in the plasma glucose and the plasma triglyceride concentrations between the groups of the db/db mouse and the +/+ mouse was defined as 100%, and the rate (%) of decrease in the plasma glucose and the plasma triglyceride concentrations of the group to which the test compound was administered was calculated. As a result, when the test compound was administered at a dose of 3.2 mg/kg, plasma glucose decreasing activity was 19%, while TG concentration-decreasing activity was 9%.

### INDUSTRIAL APPLICABILITY

Novel indole derivatives and their pharmaceutically acceptable salts are provided. These compounds and their pharmaceutically acceptable salts have blood sugar level-depressing activity or PDE5-inhibiting activity, and are useful for preventing and treating impaired glucose tolerance, diabetes (type II diabetes), diabetic complications (e.g., diabetic gangrene, diabetic arthropathy, diabetic osteopenia, diabetic glomerulosclerosis, diabetic nephropathy, diabetic dermatopathy, diabetic neuropathy, diabetic cataract, diabetic retinopathy, etc.), syndrome of insulin resistance (e.g., insulin receptor disorders, Rabson-Mendenhall syndrome, leprechaunism, Kobberling-Dunnigan syndrome, Seip syndrome, Lawrence syndrome, Cushing syndrome, acromegaly, etc.), polycystic ovary syndrome, hyperlipidemia, atherosclerosis, cardiovascular disorders (e.g., stenocardia, cardiac failure, etc.), hyperglycemia(e.g., abnormal saccharometabolism such as feeding disorders, etc.), hypertension, pulmonary hypertension, congestive heart failure, glomerulopathy (e.g., diabetic glomerulosclerosis, etc.), tubulointerstitial disorders (e.g., renopathy induced by FK506, cyclosporin, etc.), renal failure, angiostenosis (e.g., after percutaneous arterioplasty), distal angiopathy, cerebral apoplexy, chronic reversible obstructions (e.g., bronchitis, asthma (chronic asthma, allergic asthma), etc.), autoimmune diseases, allergic rhinitis, urticaria, glaucoma, diseases characterized by enteromotility disorders (e.g., hypersensitive enteropathy syndrome, etc.), impotence (e.g., organic impotence, psychic impotence, etc.), nephritis, cachexia(e.g., progressive weight loss due to the lipolysis, myolysis, anemia, edema, anorexia, etc. associated with chronic diseases such as cancer, tuberculosis, endocrine disorder, AIDS, etc.), pancreatitis, or restenosis after PTCA.

## Claims

1. An indole derivative represented by formula (I) or a salt thereof: 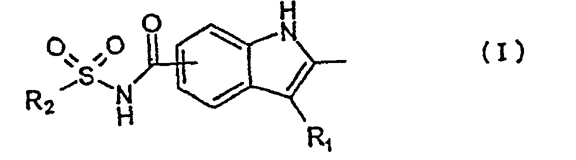 wherein R₁ represents an aryl lower alkyl group, said aryl group may be substituted with one or more groups selected from the group consisting of a halogen atom, an aryl group, a heterocyclic group, an aryl lower alkyl group, an aryl lower alkenyl group, a halo-lower alkyl group, a lower cycloalkyl-lower alkoxy group, a lower cycloalkoxy-lower alkyl group, an aryl lower alkynyl group, an aryloxy lower alkyl group, an aryl lower alkoxy group, a lower alkylthio group, a lower alkoxy group, and an alkenyl group; and R₂ represents a lower alkyl group, a lower alkenyl group, an aryl group, or a heterocyclic group, each of which may be substituted with a hydrogen atom, a lower alkyl group, a lower alkenyl group, or an aryl group.

2. The indole derivative or a salt thereof according to claim 1, wherein R₁ is a halo-aryl lower alkyl group, said aryl group may be substituted with a halo-lower alkyl group, a lower cycloalkyl lower alkoxy group, a lower cycloalkoxy lower alkyl group, an aryl lower alkynyl group, an aryloxy lower alkyl group, a lower alkylthio group, a lower alkoxy group, or a lower alkenyl group.

3. The indole derivative or a salt thereof according to claim 1, wherein said derivative is selected from the group consisting of 3-(2-chloro-4-(t-butylthio)benzyl)-2-methyl-5-(1-pentanesulfonylcarbamoyl)indole, 3-(2-chloro-4-(t-butylthio)benzyl)-2-methyl-5-(4-methylbenzene)sulfonylcarbamoyl)indole, 3-(2-chloro-4-iodo-benzyl)-2-methyl-5-(1-pentanesulfonylcarbamoyl)indole, 3-(2-chloro-4-iodobenzyl)-2-methyl-5-((4-methyl-benzene)sulfonylcarbamoyl)indole, 3-(2-chloro-4-(phenylethynyl)benzyl)-2-methyl-5-1(1-pentanesulfonylcarbamoyl)indole, 3-(2-chloro-4-(phenylethynyl)benzyl)-2-methyl-5-((4-methylbenzene)sulfonylcarbamoyl)- indole, 3-(2-chloro-4-(2-phenylethenyl)benzyl)-2-methyl-5-((4-methylbenzene)sulfonylcarbamoyl)indole, 3-(2-chloro-4-(2-phenylethenyl)benzyl)-2-methyl-5-(1-pentanesulfonylcarbamoyl)indole, 3-(2-chloro-4-(2-phenylethyl)benzyl)-2-methyl-5-((4-methylbenzene)sulfonylcarbamoyl)indole, 3-(2-chloro-4-(benzyloxy)-benzyl)-2-methyl-5-((4-methylbenzene)sulfonylcarbamoyl)indole, 3-(2-chloro-4-(cyclohexylmethyloxy)benzyl)-2-methyl-5-((4-methylbenzene)sulfonylcarbamoyl)indole, 3-(2-chloro-4-phenylbenzyl)-5-((5-chloro-2-thiophenesulfonyl)carbamoyl)-2-methylindole, 3-(2-chloro-4-phenylbenzyl)-5-((5-bromo-2-thiophenesulfonyl)carbamoyl)-2-methylindole, 3-(2-chloro-4-phenylbenzyl)-2-methyl-5-(4-pentenesulfonylcarbamoyl)indole, 3-((1-bromonaphthalen-2-yl)methyl)-5-((5-chloro-2-thiophenesulfonyl)-carbamoyl)-2-methylindole, 3-((1-bromonaphthalen-2-yl)methyl)-5-((5-bromo-2-thiophenesulfonyl)carbamoyl)-2-methylindole, 3-(4-bromo-2-chlorobenzyl)-2-methyl-5-((4-methylbenzene)sulfonylcarbamoyl)indole, 3-(4-bromo-2-chlorobenzyl)-2-methyl-5-((4-vinylbenzene)sulfonylcarbamoyl)indole, 3-(4-bromo-2-chlorobenzyl)-2-methyl-5-((2-phenylethenyl)sulfonylcarbamoyl)indole, 3-(4-bromo-2-chlorobenzyl)-2-methyl-5-((1-pentene)sulfonylcarbamoyl)indole, 3-(4-bromo-2-chlorobenzyl)-5-((5-bromo-2-thiophenesulfonyl)carbamoyl)-2-methylindole, 3-(4-bromo-2-chlorobenzyl)-2-methyl-5-(4-pentenesulfonylcarbamoyl)indole, 5-((5-chloro-2-thiophenesulfonyl)carbamoyl)-3-(2,4-dichlorobenzyl)-2-methylindole, 5-((5-bromo-2-thiophenesulfonyl)-carbamoyl)-3-(2,4-dichlorobenzyl)-2-methylindole, 3-(2-chloro-4-(trifluoromethyl)benzyl)-2-methyl-5-(1-pentanesulfonylcarbamoyl)indole, 3-(2-chloro-4-(trifluoromethyl)benzyl)-2-methyl-5-(4-methylbenzenesulfonylcarbamoyl)indole, 3-(2-chloro-4-(trifluoromethyl)benzyl)-2-methyl-5-((5-chloro-2-thiophenesulfonyl)carbamoyl)indole, 3-(2-chloro-4-(trifluoromethyl)-benzyl)-2-methyl-5-((5-bromo-2-thiophenesulfonyl)carbamoyl)-indole, 3-(2-chloro-4-(trifluoromethyl)benzyl)-2-methyl-5-((4-vinylbenzene)sulfonylcarbamoyl)indole, 3-(2-chloro-4-(trifluoromethyl)benzyl)-2-methyl-5-((2-phenylethenyl)sulfonylcarbamoyl) indole, 3-(2-chloro-4-(trifluoromethyl)benzyl)-2-methyl-5-((1-pentene)sulfonylcarbamoyl)indole, 3-(2-chloro-4-(phenoxymethyl)benzyl)-2-methyl-5-(1-pentanesulfonylcarbamoyl)indole, 3-(2-chloro-4-(phenoxymethyl)benzyl)-2-methyl-5-(4-methylbenzenesulfonylcarbamoyl)indole, 3-(2-chloro-4-(cyclohexyloxymethyl)benzyl)-2-methyl-5-(1-pentanesulfonylcarbamoyl)indole, 3-(2-chloro-4-(cyclohexyloxymethyl)benzyl)-2-methyl-5-(4-methylbenzenesulfonylcarbamoyl)indole, 3-(2-chloro-4-ethoxybenzyl)-2-methyl-5-(4-methylbenzenesulfonylcarbamoyl)indole, 3-(2-chloro-4-ethoxybenzyl)-2-methyl-5-(1-pentanesulfonylcarbamoyl)indole, 3-(2-chloro-4-(thiophen-2-yl)benzyl)-2-methyl-5-(4-methylbenzenesulfonylcarbamoyl)indole, 3-(2-chloro-4-(thiophen-2-yl-)benzyl)-2-methyl-5-(1-pentanesulfonylcarbamoyl)indole, 3-(2-chloro-4-(furan-2-yl)benzyl)-2-methyl-5-(1-pentanesulfonylcarbamoyl)indole, 3-(2-chloro-4-(furan-2-yl)benzyl)-2-methyl-5-(4-methylbenzenesulfonylcarbamoyl)indole, 3-(2-chloro-4-(1-hexen-2-yl)benzyl)-2-methyl-5-(4-methylbenzenesulfonylcarbamoyl)indole, 3-(2-chloro-4-(1-hexen-1-yl)benzyl)-2-methyl-5-(4-methylbenzenesulfonylcarbamoyl)indole, 3-(2-chloro-4-(1-hexen-2-yl)benzyl)-2-methyl-5-(1-pentanesulfonylcarbamoyl)indole, and 3-(2-chloro-4-(1-hexen-1-yl)benzyl)-2-methyl-5-(1-pentanesulfonylcarbamoyl)indole.

4. A pharmaceutical composition which comprises, as an active ingredient, the indole derivative or a salt thereof according to any one of claims 1 to 3.

5. A method of producing the indole derivative of any one of claims 1 to 3, the method comprising the steps of:
(a) reacting a compound of formula (2): 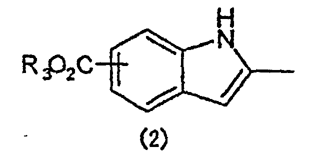 wherein R₃ represents a lower-alkyl group, with haloid or silane, and aldehyde corresponding to R₁ (R₁ has the same meaning as above );
(b) hydrolyzing a compound of formula (3) obtained in step (a): 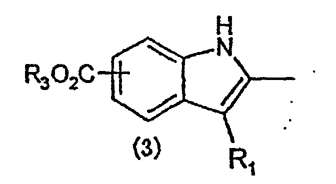 wherein R₁ has the same meaning as above; and
(c) reacting a carboxyl group-activating agent and subsequently sulfonamide with a compound of formula (4) obtained in step (b): 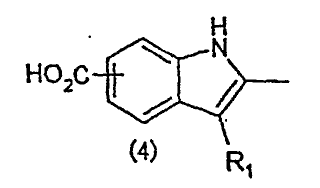 wherein R₁ has the same meaning as above.

6. A method of producing the indole derivative of any one of claims 1 to 3, the method comprising the steps of:
(a) reacting a compound of formula (2): 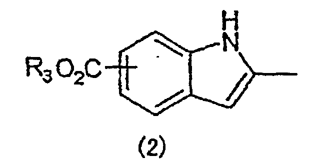 wherein R₃ represents a lower-alkyl group, with haloid or silane, and aldehyde corresponding to R₁ (R₁ has the same meaning as above );
(b) hydrolyzing a compound of formula (3) obtained in step (a): 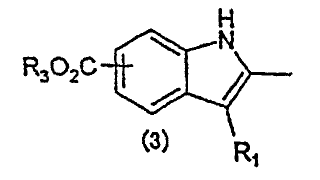 wherein R₁ has the same meaning as above;
(g) reacting a halogenating agent with a compound of formula (4) obtained in step (b): 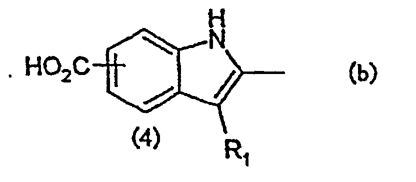 wherein R₁ has the same meaning as above; and
(h) reacting sulfonamide with a compound of formula (8) obtained in step (g): 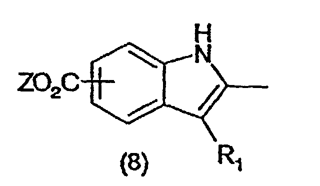 wherein z represents a halogen atom and R₁ has the same meaning as above.

7. A method of producing the indole derivative of any one of claims 1to 3, the method comprising the steps of:
(a) reacting a compound of formula (2): 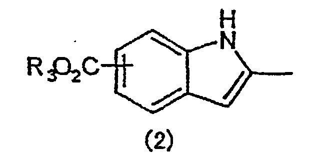 wherein R₃ represents a lower-alkyl group, with haloid or silane, and aldehyde corresponding to R₁ (R₁ has the same meaning as above );
(b) hydrolyzing a compound of formula (3) obtained in step (a): 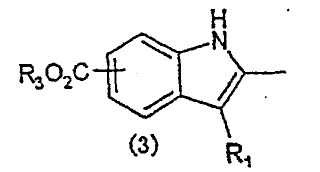 wherein R₁ has the same meaning as above;
(g) reacting a halogenating agent with a compound of formula (4) obtained in step (b): 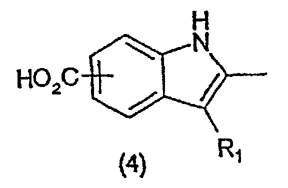 wherein R₁ has the same meaning as above;
(i) reacting ammonia or aqueous ammonia with a compound of formula (8) obtained in step (g): 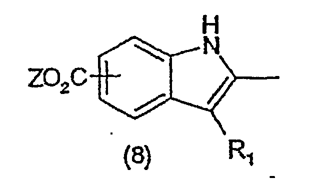 wherein Z represents a halogen atom and R₁ has the same meaning as above; and
(j) reacting sulfonylhalide to a compound of formula (9) obtained in step (i): 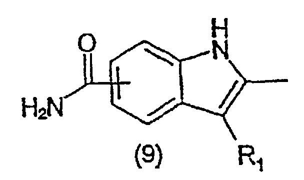 wherein R₁ has the same meaning as above.

8. Use of the indole derivative or a salt thereof according to any one of claims 1 to 3 for the manufacture of a pharmaceutical composition for preventing and treating impaired glucose tolerance, diabetes, diabetic complications, syndrome of insulin resistance, polycystic ovary syndrome, hyperlipidemia, atherosclerosis, cardiovascular disorders, hyperglycemia, hypertension, pulmonary hypertension, congestive heart failure, glomerulopathy, tubulointerstitial disorders, renal failure, angiostenosis, distal angiopathy, cerebral apoplexy, chronic reversible obstructions, autoimmune diseases, allergic rhinitis, urticaria, glaucoma, diseases characterized by enteromotility disorders, impotence, nephritis, cachexia, pancreatitis, or restenosis after PTCA.
